(19) Europäisches Patentamt
European Patent Office
Office européen des brevets

(11) **EP 4 745 974 A1**

(12) **EUROPEAN PATENT APPLICATION**

(43) Date of publication:
**20.05.2026 Bulletin 2026/21**

(21) Application number: **25215665.8**

(22) Date of filing: **13.11.2025**

(51) International Patent Classification (IPC):
**G16C 20/20** (2019.01)   **H01J 49/00** (2006.01)

(52) Cooperative Patent Classification (CPC):
**G16C 20/20; H01J 49/0036**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **14.11.2024 US 202418947531**

(71) Applicant: **HighChem s.r.o.
821 09 Bratislava (SK)**

(72) Inventor: **LUTISAN, Juraj
821 09 Bratislava (SK)**

(74) Representative: **Boult Wade Tennant LLP
Salisbury Square House
8 Salisbury Square
London EC4Y 8AP (GB)**

(54) **SIMILARITY ESTIMATION AMONG SPECTRAL DATASETS USING SPECTRAL BREAKDOWN CURVES**

(57) Embodiments described herein relate to evaluating similarity between spectral datasets. A system can comprise a memory that stores, and a processor that executes, computer executable components, which can comprise an identifying component that identifies a first set of spectral breakdown data for a first compound, corresponding to first ion activation energies that comprise a target ion activation energy omitted from prior spectrometry measurement of the first compound, wherein the identifying component further identifies a second set of spectral breakdown data for a second compound, corresponding to second ion activation energies, and a comparing component that executes a comparison of the first set of spectral breakdown data to the second set of spectral breakdown data at the target ion activation energy, resulting in a target similarity value defining a similarity between the first set of spectral breakdown data and second set of spectral breakdown data at the target ion activation energy.

FIG. 1

**Description**

CROSS REFERENCE TO RELATED APPLICATION

This application claims the benefit of U.S. Non-Provisional Application S/N 18/947,531, filed November 11, 2024, which is incorporated by reference in its entirety.

BACKGROUND

Comparison of spectral data from one or more chemical structure measurement devices can be a complicated and time-intensive process, based on data obtained over a plurality of ion activation energies, retention times, repeated operations, etc., thus occupying the chemical structure measurement devices and/or taking user entity time away from other tasks. Likewise, comparison of data from a same and/or plural instruments can be prefaced on input of data obtained at a large plurality of ion activation energies to allow for at least a partially comprehensive understanding of overall spectral and/or breakdown data for a target compound.

SUMMARY

The following presents a summary to provide a basic understanding of one or more example embodiments described herein. This summary is not intended to identify key or critical elements, and/or to delineate scope of particular embodiments or scope of claims. Its sole purpose is to present concepts in a simplified form as a prelude to the more detailed description that is presented later. In one or more example embodiments, systems, computer-implemented methods, apparatuses and/or computer program products described herein can provide a plug-and-play process for using data generated by a measurement instrument (also herein referred to as a measurement device) to calibrate, normalize and/or compare measurement instrument output data in a time efficient and automatic manner.

In accordance with an embodiment, a system can comprise a memory that stores computer executable components, and a processor that executes the computer executable components. The computer executable components can comprise an identifying component that identifies a first set of spectral breakdown data for a first compound, corresponding to first ion activation energies that comprise a target ion activation energy omitted from prior spectrometry measurement of the first compound, wherein the identifying component further identifies a second set of spectral breakdown data for a second compound, corresponding to second ion activation energies, and a comparing component that executes a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

In accordance with another embodiment, a computer-implemented method can comprise identifying, by a system operatively coupled to a processor, a first set of spectral breakdown data for a first compound, corresponding to first ion activation energies that comprise a target ion activation energy omitted from prior spectrometry measurement of the first compound, identifying, by the system, a second set of spectral breakdown data for a second compound, corresponding to second ion activation energies, and executing, by the system, a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

In accordance with still another embodiment, a computer program product facilitates a process for evaluating similarity between spectral datasets, the program instructions executable by a processor to cause the processor to identify, by the processor, a first set of spectral breakdown data for a first compound, corresponding to first ion activation energies that comprise a target ion activation energy omitted from prior spectrometry measurement of the first compound, identify, by the processor, a second set of spectral breakdown data for a second compound, corresponding to second ion activation energies, and execute, by the processor, a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

The one or more example embodiments described herein can be implemented within, in connection with and/or coupled to a chemical structure measurement device, such as a scientific measurement device.

The one or more example embodiments disclosed herein can be applied on a plug-and-play basis to a measurement device, plural measurement devices, a same measurement device using plural exchangeable components, etc. for calibration, normalization and/or comparison of output data relative to unknown, known and/or standard data. The frameworks described herein can be performed in a time efficient and at least partially automatic manner, thereby increasing device use time and/or reducing user entity interaction for pre-experiment and/or post-experiment processes.

The one or more example embodiments described herein can be employed to approximate spectral data corresponding to ion activation energies not specifically employed during spectrometric operations. This can be accomplished by employing approximating functions to breakdown curve data corresponding to one or more fragment ions fragmented from a compound at other ion activation energies. In this way, comparison between breakdown curve data for different compounds, different devices, same compound but different devices, etc. can be made over a range of ion activation energies without directly obtaining spectral data at all ion activation energies of the range of ion activation energies. As a result, the one or more embodiments described herein can reduce a number of fragmentation runs to be performed at a measurement device to obtain initial mass spectrometry data from which the breakdown data is generated.

Moreover, based on the use of approximated spectral breakdown data, resulting from the approximation of the breakdown curve data, a more comprehensive understanding of the breakdown data can be obtained over a range of ion activation energies, as compared to existing frameworks. This can allow for identification of local minima or local maxima of quantified similarity data between and/or among two or more sets of spectral breakdown data, with at least one of the sets resulting from the aforementioned approximation process. Optionally, all sets of spectral breakdown data being compared can result from the aforementioned approximation process.

BRIEF DESCRIPTION OF THE DRAWINGS

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.

FIG. 1 illustrates a block diagram of an example, non-limiting system that can facilitate a process for measurement device data comparison, in accordance with one or more example embodiments described herein.

FIG. 2 illustrates a block diagram of another example, non-limiting system that can facilitate a process for measurement device data comparison, in accordance with one or more example embodiments described herein.

FIG. 3 illustrates an example graph of example breakdown curve data, in accordance with one or more example embodiments described herein.

FIG. 4 illustrates a set of example graphs of example approximations of the example breakdown curve data of FIG. 3, in accordance with one or more example embodiments described herein.

FIG. 5 illustrates an example graph of approximated spectral breakdown data corresponding to FIG. 3, and a target spectrum generated based on the approximated spectral breakdown data, in accordance with one or more example embodiments described herein.

FIG. 6 illustrates an example graph of comparison data resulting from comparison of sets of spectral breakdown data, including the spectral breakdown data of FIG. 5, for a single fragmentation ion, in accordance with one or more example embodiments described herein.

FIG. 7 illustrates an example graph of comparison data resulting from comparison of sets of spectral breakdown data, including the spectral breakdown data of FIG. 5, for plural fragmentation ions, in accordance with one or more example embodiments described herein.

FIG. 8 illustrates a set of graphs schematically illustrating normalization of ion activation energy and transformation of spectral breakdown data from different measurement instruments, in accordance with one or more example embodiments described herein.

FIG. 9 illustrates an example node-based graph employing total spectra similarity to visually link nodes as a set of edges, and a summary of processes performed by the non-limiting system of FIG. 2, in accordance with one or more example embodiments described herein.

FIG. 10 illustrates a flow diagram of one or more processes that can be performed by the non-limiting system of FIG. 1, in accordance with one or more example embodiments described herein.

FIG. 11 illustrates another flow diagram of one or more processes that can be performed by the non-limiting system of FIG. 2, in accordance with one or more example embodiments described herein.

FIG. 12 illustrates a continuation of the flow diagram of FIG. 11 of one or more processes that can be performed by the non-limiting system of FIG. 2, in accordance with one or more example embodiments described herein.

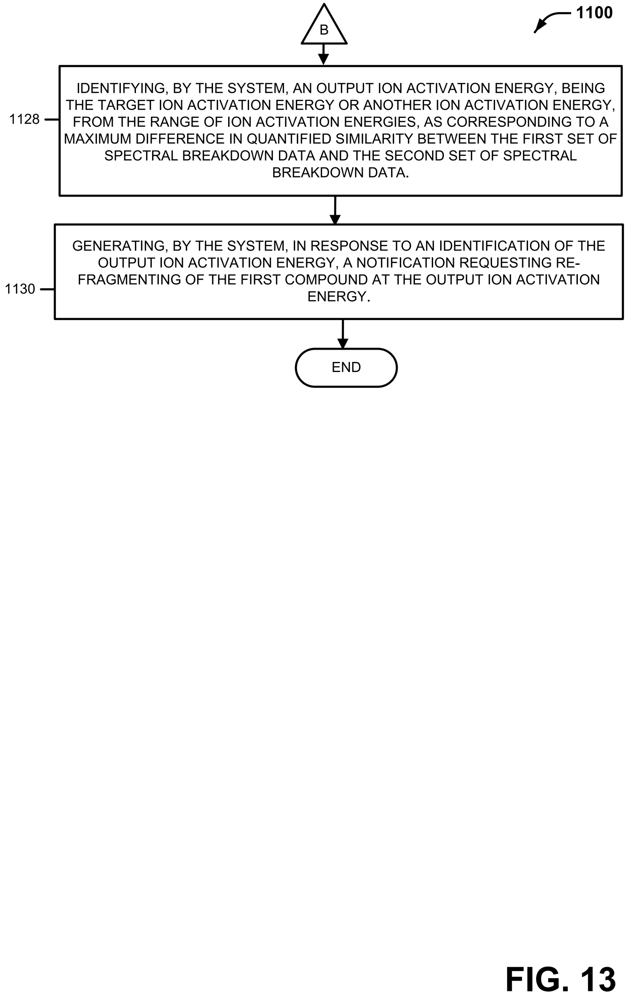

FIG. 13 illustrates a continuation of the flow diagram of FIG. 12 of one or more processes that can be performed by the non-limiting system of FIG. 2, in accordance with one or more example embodiments described herein.

FIG. 14 illustrates a block diagram of an example operating environment into which embodiments of the subject matter described herein can be incorporated.

FIG. 15 illustrates an example schematic block diagram of a computing environment with which the subject matter described herein can interact and/or be implemented at least in part.

DETAILED DESCRIPTION

The following detailed description is merely illustrative and is not intended to limit embodiments and/or application or utilization of embodiments. Furthermore, there is no intention to be bound by any expressed or implied information presented in the preceding Summary section, or in the Detailed Description section.

Turning first to the subject of chemical structure measurement devices generally, such measurement devices can comprise, but are not limited to spectrometry devices, chromatography devices, combination spectrometry and chromatography devices, etc. Output from such devices can be measurement data that comprises intensities, mass-to-charge ratios, etc. of compounds analyzed and/or of ions fragmented from the compounds during analysis. One such type of measurement data can be mass spectrometry data resulting from operation of a mass spectrometry device. To allow for comparison of such spectral data from plural compounds and/or plural devices, and/or against one or more known and/or standardized datasets, it can be advantageous to acquire a plurality of sub-datasets, each at a different ion activation energy. However, this can be tedious, inefficient and/or time consuming. Even in view of such extended data gathering, data representing local minima or local maxima of similarity between datasets can be missed, omitted and/or comprise an error and/or outlier, etc.

Accordingly, to account for one or more of these deficiencies, the one or more embodiments described herein can provide a process for employing approximated spectral breakdown data, based on initial mass spectrometry data (e.g., initial spectral data) that is converted to breakdown curve data and then approximated, to evaluate similarity between spectral datasets and/or compounds corresponding to the spectral datasets. As a result, a comprehensive understanding of similarity over a range of ion activation energies can be obtained, including identification of local minima and/or local maxima corresponding to quantified similarity values. Based thereon, one or more individual ion activation energies can be further evaluated, a database of total spectra similarity can be generated and/or updated, plural measurement device outputs corresponding to same or different compounds can be compared to one another, and/or different data output from same or different compounds at a single measurement device can be compared, without being limited thereto. These comprehensive evaluations can allow for efficient and/or automatic evaluations aiding in calibration, data evaluation and/or reduction in repeat operations.

Regardless of post-processing evaluation use, the one or more embodiments described herein can perform spectral data comparison, using approximated spectral breakdown data, resulting in comparison data (e.g., quantified similarity data) that can be automatically obtained in a time efficient manner taking less time, labor and/or initial data input than existing frameworks.

As used herein, the phrase "based on" should be understood to mean "based at least in part on," unless otherwise specified.

As used herein, the term "compound" can refer to a single material, multiple materials, composition, sample, solution, product, etc.

As used herein, the term "data" can comprise metadata.

As used herein, the terms "entity," "requesting entity," and "user entity" can refer to a machine, device, component, hardware, software, smart device, party, organization, individual and/or human.

One or more example embodiments are now described with reference to the drawings, where like referenced numerals are used to refer to like drawing elements throughout. In the following description, for purposes of explanation, numerous specific details are set forth in order to provide a more thorough understanding of the one or more example embodiments. It is evident in various cases, however, that the one or more example embodiments can be practiced without these specific details.

Further, it should be appreciated that the embodiments depicted in one or more figures described herein are for illustration only, and as such, the architecture of embodiments is not limited to the systems, devices and/or components depicted therein, nor to any particular order, connection and/or coupling of systems, devices and/or components depicted therein.

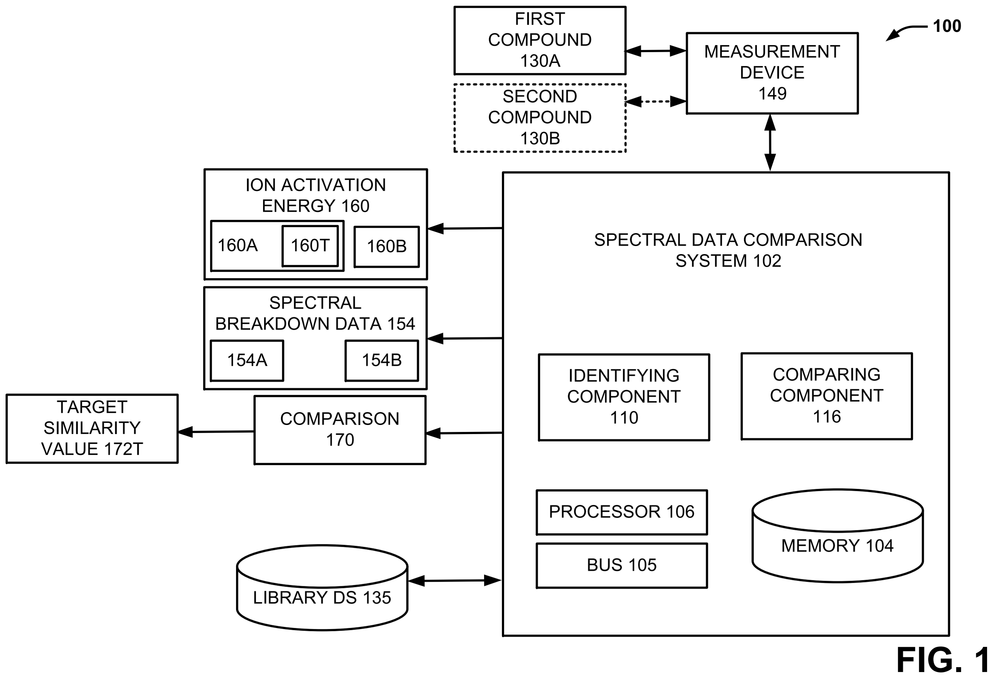

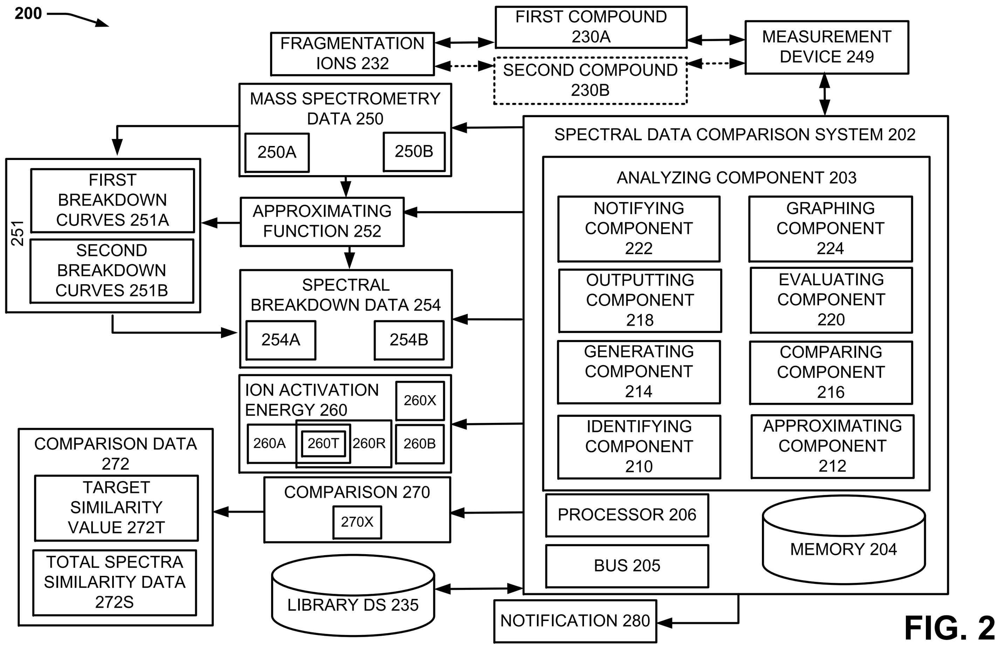

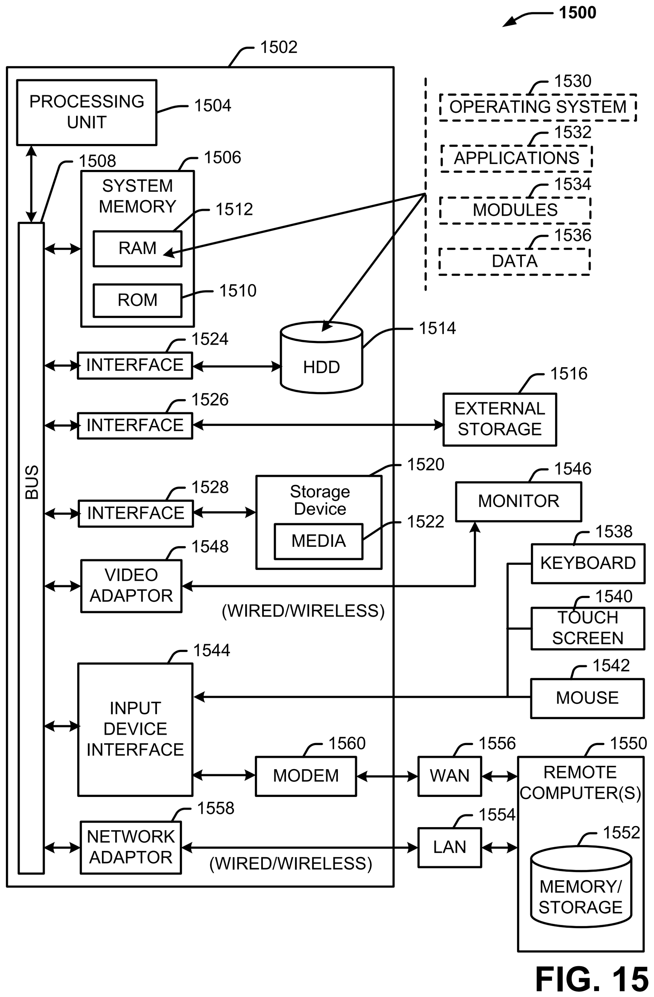

Referring now to FIGS. 1 and 2, in one or more example embodiments, the non-limiting systems 100 and/or 200 illustrated at FIGS. 1 and 2, and/or systems thereof, can further comprise one or more computer and/or computing-based elements described herein with reference to a computing environment, such as the computing environment 1500 illustrated at FIG. 15. In one or more described embodiments, computer and/or computing-based elements can be used in connection with implementing one or more of the systems, devices, components and/or computer-implemented operations shown and/or described in connection with FIGS. 1 and/or 2 and/or with other figures described herein.

Turning first to FIG. 1, the figure illustrates a block diagram of an example, non-limiting system 100 that can comprise a spectral data comparison system 102 and a library datastore (DS) 135. Optionally, the non-limiting system 100 can comprise a measurement device 149 (e.g., a spectrometry device or other scientific measurement device). In one or more other embodiments, the measurement device 149 and/or library datastore 135 can be located external to the spectral data comparison system 102 which can be communicatively coupled to the measurement device 149 and/or library datastore 135.

As used herein, both above and below throughout, the term "spectral data" can refer generally to any one or more of mass spectrometry data 250, breakdown curve data 251, and/or spectral breakdown data 154, 254.

It is noted that the spectral data comparison system 102 is only briefly detailed to provide but a lead-in to a more complex and/or more expansive spectral data comparison system 202 as illustrated at FIG. 2. That is, further detail regarding processes that can be performed by one or more example embodiments described herein will be provided below relative to the non-limiting system 200 of FIG. 2.

Still referring to FIG. 1, the spectral data comparison system 102 can generally facilitate generation and/or comparison of spectral breakdown data 154, having been approximated based on one or more approximating functions, thereby allowing for efficient understanding of local minima and/or maxima of comparison data generated based on the spectral breakdown data 154. The comparison can be executed between sets of spectral breakdown data 154A and 154B, from same or different compounds and/or same or different measurement devices, and/or where one or more sets of spectral breakdown data are from a standardized library, such as the library datastore 135.

Put another way, the spectral data comparison system 102 can generally facilitate a process to generate and/or compare spectral breakdown data 154 based on a target ion activation energy 160T not employed for spectrometric analysis that resulted in the spectral breakdown data 154.

The spectral data comparison system 102 can comprise at least a memory 104, bus 105, processor 106, identifying component 110 and/or comparing component 116. The processor 106 can be the same as the processor 1504 (FIG. 15), comprised by the processor 1504 or different therefrom. The memory 104 can be the same as the system memory 1506 (FIG. 15), comprised by the system memory 1506 or different therefrom.

Using the above-noted components, the spectral data comparison system 102 can facilitate a process to execute one or more comparisons 170 of spectral breakdown data 154, resulting in generation of one or more target similarity values 172T quantitatively expressing a similarity between different sets 154A, 154B of the spectral breakdown data 154.

Generally, the identifying component 110 can identify a first set of spectral breakdown data 154A for a first compound 130A, corresponding to first ion activation energies 160A that comprise a target ion activation energy 160T omitted from prior spectrometry measurement of the first compound 130A. That is, the prior spectrometry measurement can use a portion of the first ion activation energies 160A, but not comprising the target ion activation energy 160T. The identifying component 110 further can identify a second set of spectral breakdown data 154B for a second compound 130B, corresponding to second ion activation energies 160B.

It is noted that the first compound 130A and the second compound 130B can be the same or different compounds.

It is noted that the first ion activation energies 160A can comprise the same and/or different ion activation energies than the second ion activation energies 160B.

In one or more cases, the comparing component 116, and/or the processor 106, can make a determination of whether sufficiently expanded (e.g., approximated) data is provided to facilitate a comparison 170, such as within and/or satisfying an error threshold, and/or based on a quantity of outlier values of the spectral breakdown data 154.

The comparing component 116 can generally execute the comparison 172 of the first set of spectral breakdown data 154A to the second set of spectral breakdown data 154B at the target ion activation energy 160T, resulting in a target similarity value 172T defining a similarity between the first set of spectral breakdown data 154A and the second set of spectral breakdown data at the target ion activation energy 160T.

The identifying component 110 and/or comparing component 116 can be operatively coupled to the processor 106 which can be operatively coupled to the memory 104. The bus 105 can provide for the operative coupling. The processor 106 can facilitate execution of the identifying component 110 and/or comparing component 116. The identifying component 110 and/or comparing component 116 can be stored at the memory 104.

In general, the non-limiting system 100 can employ any suitable method of communication (e.g., electronic, communicative, internet, infrared, fiber, etc.) to provide communication between the spectral data comparison system 102 and/or any device associated with a user entity, such as the measurement device 149, such as a spectrometry device.

It is noted that one or more additional measurement devices likewise can be communicatively couplable with the non-limiting system 100 and/or comprised by the non-limiting system 100. For example, a first measurement device 149 can have performed spectrometry analysis on the first compound 130A and a second measurement device 149 can have performed spectrometry analysis on the second compound 130B. Alternatively, a same measurement device 149 can have been used for spectrometric analysis of both compounds 130A and 130B.

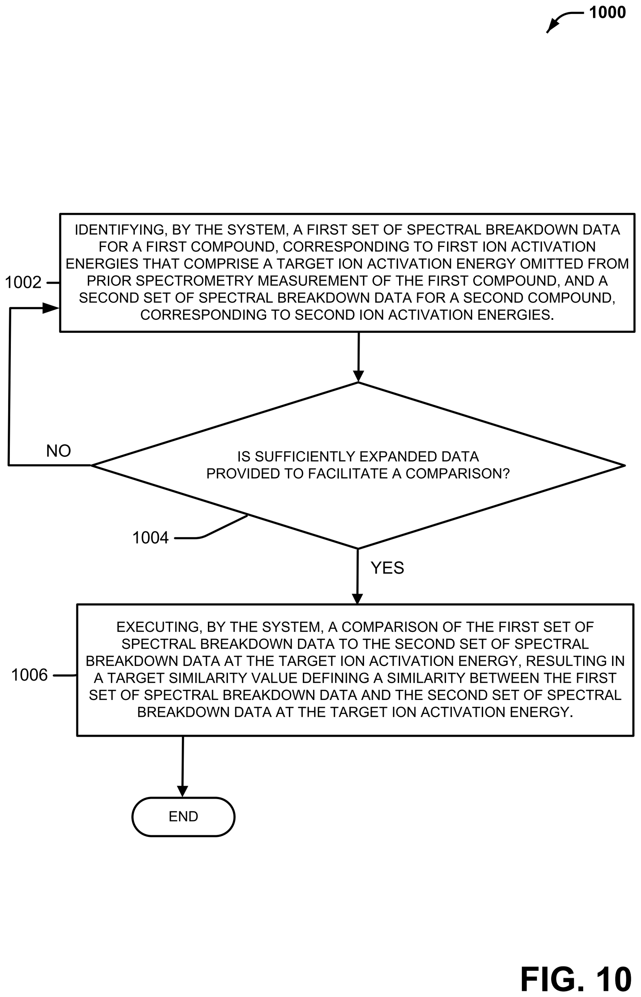

As a summary of the above-described components and functions thereof, referring next briefly to FIG. 10, illustrated is a flow diagram of an example, non-limiting method 1000 that can facilitate a process to generate and/or compare spectral breakdown data based on a target ion activation energy not employed for spectrometric analysis that resulted in the spectral breakdown data, in accordance with one or more example embodiments described herein, such as the non-limiting system 100 of FIG. 1. While the non-limiting method 1000 is described relative to the non-limiting system 100 of FIG. 1, the non-limiting method 1000 can be applicable also to other systems described herein, such as the non-limiting system 200 of FIG. 2. Repetitive description of like elements and/or processes employed in respective embodiments is omitted for sake of brevity.

At 1002, the non-limiting method 1000 can comprise identifying, by a system (e.g., identifying component 110), operatively coupled to a processor (e.g., processor 106), a first set of spectral breakdown data (e.g., first set of spectral breakdown data 154A) for a first compound (e.g., first compound 130A), corresponding to first ion activation energies (e.g., first ion activation energies 160A) that comprise a target ion activation energy (e.g., target ion activation energy 160T) omitted from prior spectrometry measurement of the first compound (e.g., first compound 130A), and a second set of spectral breakdown data (e.g., second set of spectral breakdown data 154B) for a second compound (e.g., second compound 130B), corresponding to second ion activation energies (e.g., second ion activation energies 160B).

At 1004, the non-limiting method 1000 can comprise determining, by the system (e.g., comparing component 116), whether sufficiently expanded data (e.g., spectral breakdown data 154) has been provided to facilitate a comparison (e.g., comparison 172). This can be based on such data being within and/or satisfying an error threshold, and/or based on a quantity of outlier values of the spectral breakdown data (e.g., spectral breakdown data 154). If yes, the non-limiting method 1000 can proceed to step 1006. If not, the non-limiting method can proceed back to step 1002 for additional identifying of spectral breakdown data.

At 1006, the non-limiting method 1000 can comprise executing, by the system (e.g., comparing component 116) a comparison of the first set of spectral breakdown data (e.g., first set of spectral breakdown data 154A) to the second set of spectral breakdown data (e.g., second set of spectral breakdown data 154B) at the target ion activation energy (e.g., target ion activation energy 160T), resulting in a target similarity value (e.g., target similarity value 172T) defining a similarity between the first set of spectral breakdown data (e.g., first set of spectral breakdown data 154A) and the second set of spectral breakdown data (e.g., second set of spectral breakdown data 154B) at the target ion activation energy (e.g., target ion activation energy 160T).

Turning next to FIG. 2, a non-limiting system 200 is illustrated that can comprise a spectral data comparison system 202, a measurement device 249 and a library datastore (DS) 235. Repetitive description of like elements and/or processes employed in respective embodiments is omitted for sake of brevity. Description relative to an embodiment of FIG. 1 can be applicable to an embodiment of FIG. 2. Likewise, description relative to an embodiment of FIG. 2 can be applicable to an embodiment of FIG. 1.

In one or more embodiments, the measurement device 249, such as a spectrometry device, can be separate from but communicatively couplable to the non-limiting system 200.

In one or more embodiments, one or more additional measurement devices likewise can be communicatively couplable with the non-limiting system 200 and/or comprised by the non-limiting system 200. For example, a first measurement device 249 can have performed spectrometry analysis on a first compound 230A, and a second measurement device 249 can have performed spectrometry analysis on a second compound 230B. Alternatively, a same measurement device 249 can have been used for spectrometric analysis of both compounds 230A and 230B.

In one or more embodiments, the library datastore 235 be separate from but communicatively couplable to the non-limiting system 200.

In one or more embodiments, the compounds 230A and 230B can be same or different compounds.

Generally, the spectral data comparison system 202 can facilitate generation and/or comparison of spectral breakdown data 254, having been approximated (e.g., fit) based on one or more approximating functions 252, thereby allowing for efficient understanding of local minima and/or maxima of comparison data 272 generated based on the spectral breakdown data 254. The comparison 270 can be executed between sets of spectral breakdown data 254A and 254B, from same or different compounds 230A, 230B and/or same or different measurement devices 249, and/or where one or more sets of spectral breakdown data 254A, 254B are from a standardized library, such as the library datastore 235.

Put another way, the spectral data comparison system 202 can generally facilitate a process to generate and/or compare spectral breakdown data 254 based on a target ion activation energy 260T not employed for spectrometric analysis that resulted in the spectral breakdown data 254.

One or more communications between one or more components of the non-limiting system 200 can be provided by wired and/or wireless means including, but not limited to, employing a cellular network, a wide area network (WAN) (e.g., the Internet), and/or a local area network (LAN). Suitable wired or wireless technologies for supporting the communications can include, without being limited to, wireless fidelity (Wi-Fi), global system for mobile communications (GSM), universal mobile telecommunications system (UMTS), worldwide interoperability for microwave access (WiMAX), enhanced general packet radio service (enhanced GPRS), third generation partnership project (3GPP) long term evolution (LTE), third generation partnership project 2 (3GPP2) ultra-mobile broadband (UMB), high speed packet access (HSPA), Zigbee and other 802.XX wireless technologies and/or legacy telecommunication technologies, BLUETOOTH®, Session Initiation Protocol (SIP), ZIGBEE®, RF4CE protocol, WirelessHART protocol, 6LoWPAN (Ipv6 over Low power Wireless Area Networks), Z-Wave, an advanced and/or adaptive network technology (ANT), an ultra-wideband (UWB) standard protocol and/or other proprietary and/or non-proprietary communication protocols.

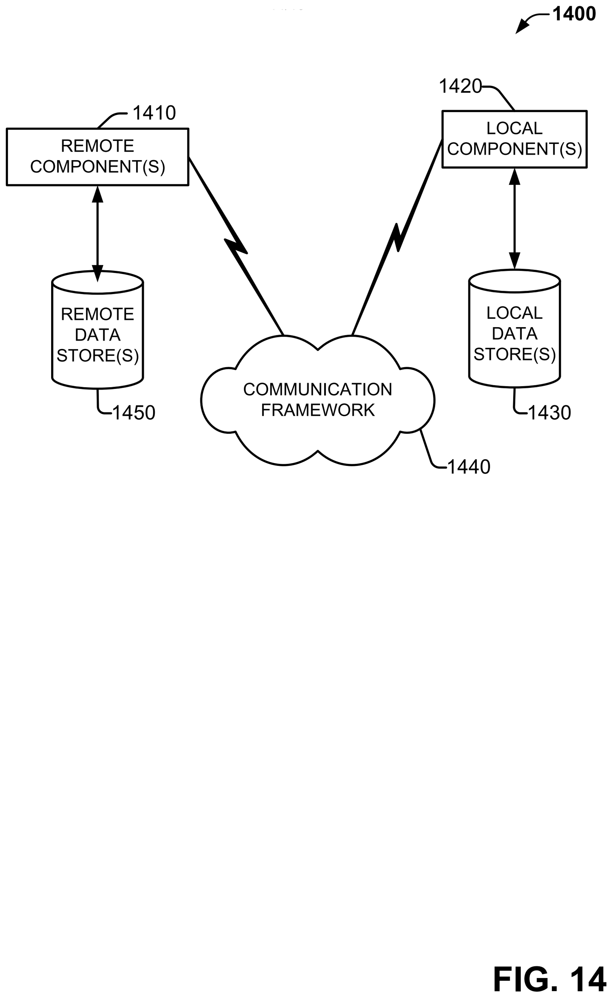

The spectral data comparison system 202 can be associated with, such as accessible via, a cloud computing environment, such as the cloud computing environment 1400 of FIG. 14.

The spectral data comparison system 202 can comprise a plurality of components. The components can comprise a memory 204, processor 206, bus 205, identifying component 210, approximating component 212, generating component 214, comparing component 216, outputting component 218, evaluating component 220, notifying component 222, and/or graphing component 224. Using these components, the spectral data comparison system 202 can facilitate a process to generate one or more target similarity values 272T upon which a determination similarity of the spectral breakdown data 254 can be made.

Discussion next turns to the processor 206, memory 204 and bus 205 of the spectral data comparison system 202. For example, in one or more example embodiments, the spectral data comparison system 202 can comprise the processor 206 (e.g., computer processing unit, microprocessor, classical processor, quantum processor and/or like processor). In one or more example embodiments, a component associated with spectral data comparison system 202, as described herein with or without reference to the one or more figures of the one or more example embodiments, can comprise one or more computer and/or machine readable, writable and/or executable components and/or instructions that can be executed by processor 206 to provide performance of one or more processes defined by such component and/or instruction. In one or more example embodiments, the processor 206 can comprise the identifying component 210, approximating component 212, generating component 214, comparing component 216, outputting component 218, evaluating component 220, notifying component 222, and/or graphing component 224.

In one or more example embodiments, the spectral data comparison system 202 can comprise the computer-readable memory 204 that can be operably connected to the processor 206. The memory 204 can store computer-executable instructions that, upon execution by the processor 206, can cause the processor 206 and/or one or more other components of the spectral data comparison system 202 (e.g., identifying component 210, approximating component 212, generating component 214, comparing component 216, outputting component 218, evaluating component 220, notifying component 222, and/or graphing component 224) to perform one or more actions. In one or more example embodiments, the memory 204 can store computer-executable components (e.g., identifying component 210, approximating component 212, generating component 214, comparing component 216, outputting component 218, evaluating component 220, notifying component 222, and/or graphing component 224).

The spectral data comparison system 202 and/or a component thereof as described herein, can be communicatively, electrically, operatively, optically and/or otherwise coupled to one another via a bus 205. Bus 205 can comprise one or more of a memory bus, memory controller, peripheral bus, external bus, local bus, quantum bus and/or another type of bus that can employ one or more bus architectures. One or more of these examples of bus 205 can be employed.

In one or more example embodiments, the spectral data comparison system 202 can be coupled (e.g., communicatively, electrically, operatively, optically and/or like function) to one or more external systems (e.g., a non-illustrated electrical output production system, one or more output targets and/or an output target controller), sources and/or devices (e.g., classical and/or quantum computing devices, communication devices and/or like devices), such as via a network. In one or more example embodiments, one or more of the components of the spectral data comparison system 202 and/or of the non-limiting system 200 can reside in the cloud, and/or can reside locally in a local computing environment (e.g., at a specified location).

In addition to the processor 206 and/or memory 204 described above, the spectral data comparison system 202 can comprise one or more computer and/or machine readable, writable and/or executable components and/or instructions that, when executed by processor 206, can provide performance of one or more operations defined by such component and/or instruction.

Discussion next turns to the additional components of the spectral data comparison system 202 (e.g., identifying component 210, approximating component 212, generating component 214, comparing component 216, outputting component 218, evaluating component 220, notifying component 222, and/or graphing component 224). As noted above, generally, the spectral data comparison system 202 can facilitate a process to generate and/or compare spectral breakdown data 254 based on a target ion activation energy 260T not employed for spectrometric analysis (e.g., by the measurement device 249) that resulted in the spectral breakdown data 254.

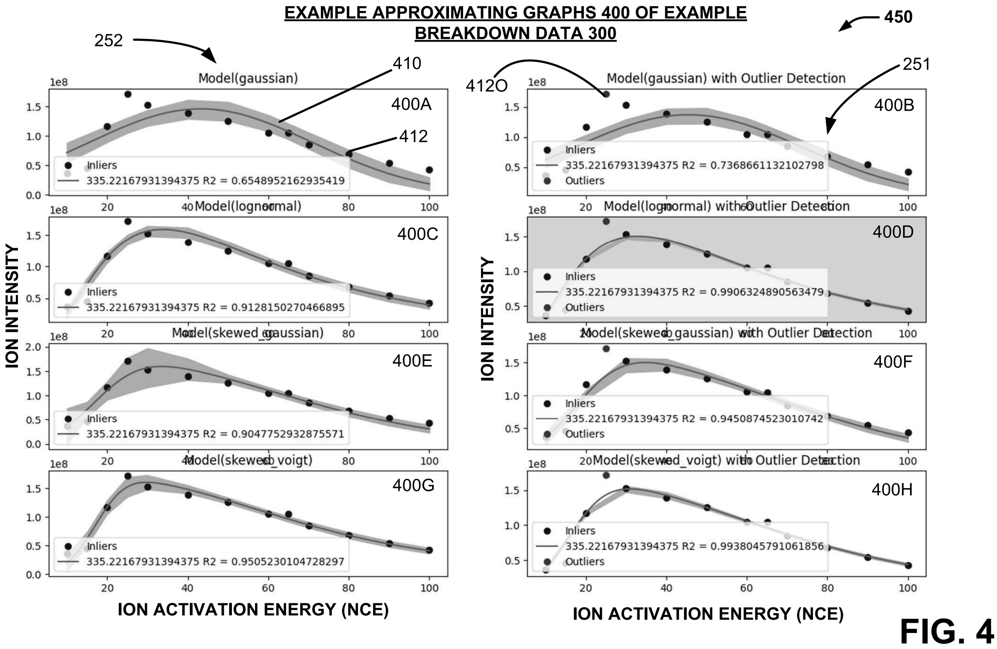

This process can be broken down into a set of processes including, but not limited to obtaining and approximating breakdown curve data 251 based on mass spectrometry data 250, executing a comparison 270 of sets of spectral breakdown data 254 resulting from an approximating 450 (FIG. 4) of the breakdown curve data 251, and evaluation of comparison data 272 output from the comparison 270.

First, it is noted that in one or more example embodiments, the identifying component 210, approximating component 212, generating component 214, comparing component 216, outputting component 218, evaluating component 220, notifying component 222, and/or graphing component 224 can be implemented independently, without one or more other of the identifying component 210, approximating component 212, generating component 214, comparing component 216, outputting component 218, evaluating component 220, notifying component 222, and/or graphing component 224. Additionally and/or alternatively, the identifying component 210, approximating component 212, generating component 214, comparing component 216, outputting component 218, evaluating component 220, notifying component 222, and/or graphing component 224 can be comprised by a high-level analyzing component 203, one or more of the below-described functions of the identifying component 210, approximating component 212, generating component 214, comparing component 216, outputting component 218, evaluating component 220, notifying component 222, and/or graphing component 224 can be performed by the high-level analyzing component 203, and/or the identifying component 210, approximating component 212, generating component 214, comparing component 216, outputting component 218, evaluating component 220, notifying component 222, and/or graphing component 224 can be omitted with the high-level analyzing component 203 performing one or more of the below-described functions of the one or more omitted identifying component 210, approximating component 212, generating component 214, comparing component 216, outputting component 218, evaluating component 220, notifying component 222, and/or graphing component 224.

As noted above, a first set of one or more processes can comprise obtaining and approximating breakdown curve data 251 based on mass spectrometry data 250.

Turning first to the identifying component 210, this component can generally acquire (e.g., obtain, locate, identify, request, download, etc.) a first set of mass spectrometry data 250A. In one or more cases, the identifying component 210 can further acquire a second set of mass spectrometry data 250B. However, in one or more other cases, the identifying component 210 can instead later acquire a second set of spectral breakdown data 254, having been already generated from the second set of mass spectrometry data 250B.

That is, the spectral data comparison system 202 can facilitate both generation of the first set of spectral breakdown data 254A from the first set of mass spectrometry data 250A and generation of the second set of spectral breakdown data 254B from the second set of mass spectrometry data 250B. Alternatively, the spectral data comparison system 202 can facilitate only generation of the first set of spectral breakdown data 254A from the first set of mass spectrometry data 250A.

Using the mass spectrometry data 250 acquired (e.g., first set of mass spectrometry data 250A and, optionally, the second set of mass spectrometry data 250B), the approximating component 212 can generate first breakdown curves 251A and, optionally, second breakdown curves 251B.

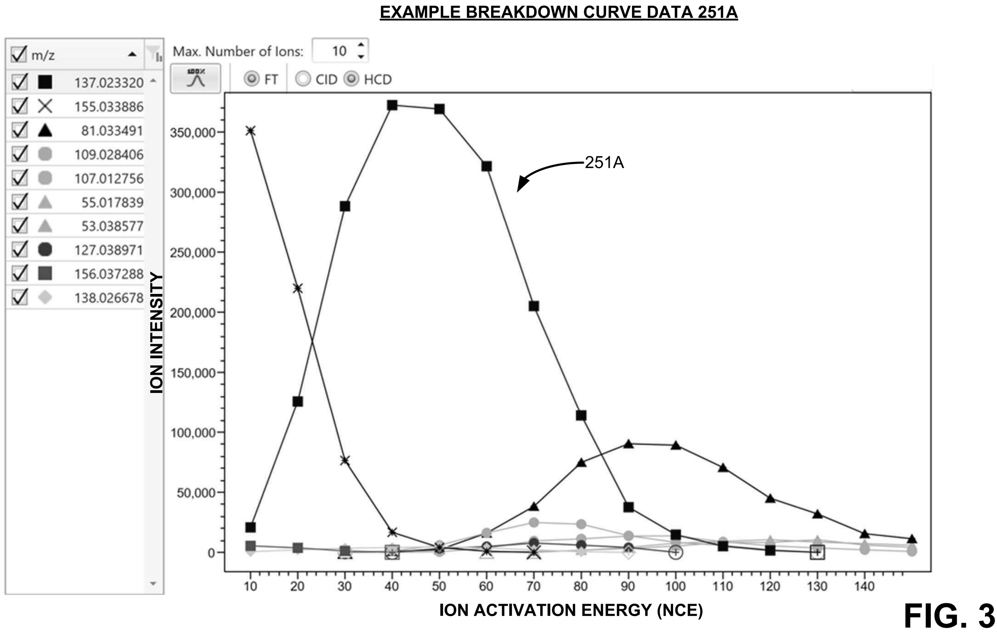

For example, referring briefly to FIG. 3, but also still to FIG. 2, illustrated at FIG. 3 is an example graph 300 of example breakdown curve data 251A.

In general, the graph 300 comprises a set of breakdown curves 251A having been generated from the mass spectrometry data 250.

A set of breakdown curves (BDC) can provide a complete description of the mass spectrometry (MS) spectra (e.g., $MS^n$, wherein n > 1) for an ion activation energy range. The BDC can be constructed from set of experiments where $MS^n$ spectra for identical precursor (e.g. $[M+H]^+$ ) and/or for identical ion activation condition (e.g. ion activation energy such as collision-induced dissociation or CID) are measured for different energy, e.g. CID 10, CID 20, CID 30, CID 40, CID 50, CID 60, CID 70, CID 80, CID 90, CID 100.

Note that different precursor or ion activation type (CID, higher energy collision dissociation or HCD, ultra violet photo dissociation or UVPD, etc.) cannot be mixed to one set of BDC curves. That is, Due to different mechanisms, it is impossible to mix different ion activation types into one set of BDC curves. Accordingly, FIG. 3 cannot contain different ion activation types. Rather, it shows the dependency of intensities on ion activation energy for a single ion activation type. Each ion activation type employs a separate figure.

It is noted that M represents the mass of the analyzed un-ionized compound (the whole molecule), and H indicates that a hydrogen cation is attached to the molecule. The resulting ion is denoted as [M+H]+.

For example, the approximating component 212 can identify a set of individual m/z ions from the mass spectrometry data 250, and then can construct one BDC curve per m/z ion, collecting ion intensity of the ion for each ion activation energy at which the mass spectrometry data 250 was obtained. As a result, the BDC can be a set of scatter graphs for all individual ions.

Note that depending on the detector employed, the units of ion intensity can be amperes (e.g., picoamperes or nanoamperes) or counts per seconds. In one or more other embodiments, units of ion intensity can be not be shown on the graph. That is, a common practice can be to use relative intensity, where the highest peak is assigned an intensity of 100%.

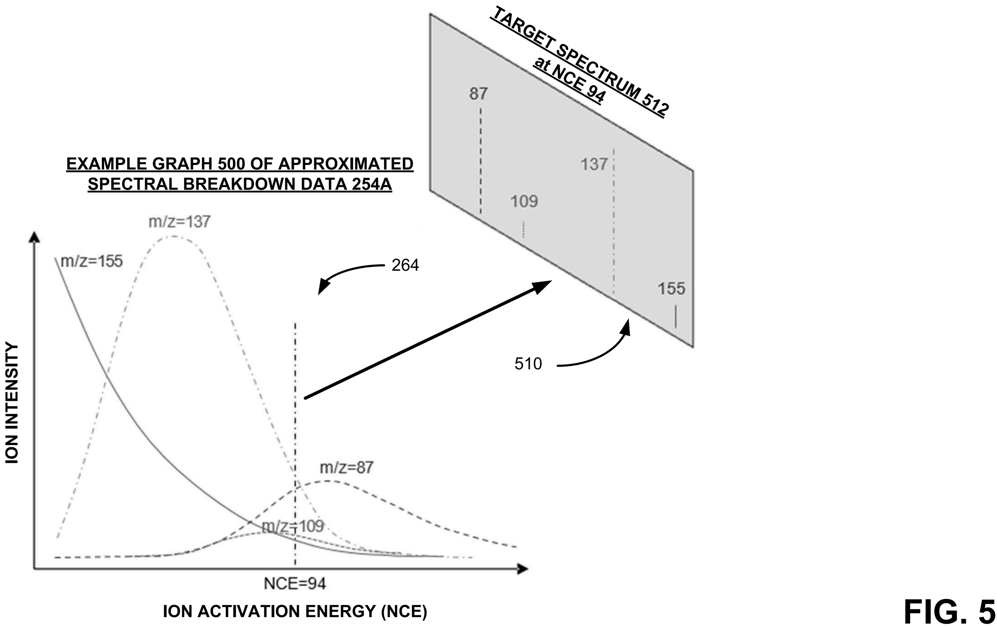

Note also that the non-fragmented ion, referred to as the precursor ion (e.g., [M+H]+ or [M-H]- for MS2 nodes), is represented in the graph of BDC curves. For instance, the curve labeled as m/z=155 in FIG. 5 represents these precursor ions. At the lowest energy levels, the abundance of this unfragmented ion is the highest. As the energy increases, its abundance decreases, while the abundance of ions resulting from fragmentation increases.

In one or more other embodiments, the BDC data 251 can be directly obtained (e.g., by the identifying component 210) without generation of the BDC 251 by the approximating component 212. Note that the BDC data 251 obtained still can be non-approximated (e.g., non-expanded, non-fit, non-interpolated, etc.).

BDC can be employed to characterize and/or differentiate even structurally similar compounds as positional isomers, as a tool to verify liquid chromatography - mass spectrometry/mass spectrometry (LC-MS/MS) methods regarding consistent fragmentation characteristics between sample sources, native analytes and/or isotope-labeled counterparts, without being limited thereto. In existing frameworks, only visual analysis of BDC is performed and a generalizing mathematical apparatus for their processing, and particularly approximating, is not employed.

Referring still to FIG. 3, it is noted that the ion activation energy at the x-axis of graph 300 is identified as normalized collision energy (NCE). That is, ion activation energy is a general term that can be expressed in volts. NCE can be used for specific ion activation techniques like CID (collision-induced dissociation) or HCD (higher-energy collisional dissociation). Ion activation energy can be absolute (in volts) or normalized. Both values (e.g., absolute and normalized) can be stored in a spectrum. The relationship between these values can defined by the software managing, employed by, employing, and/or comprised by a measurement instrument (e.g., spectrometry instrument).

Put another way, each line of the graph 300 represents a different fragmentation aspect, such as an ion fragmented from the compound 230A or can represent the remaining compound 230A (minus the one or more ions fragmented therefrom). Each point at the lines represents an ion activation energy vs. ion intensity for a single spectrum. That is, the initial mass spectrometry data 250A can have been generated by spectrometric analysis at each of 15 different ion activation energies (NCE from 10 to 150). The estimated, rough and/or straight lines connecting the points merely trace/connect one point to next adjacent point and so on. In the graph 300, the lines can comprise outlier data points and do not provide an accurate representation of data in between the data points exhibited at graph 300. That is, the breakdown curve data 251 has not yet been expanded (e.g., fit) and/or interpolated.

Accordingly, turning next to the approximating component 212 and to FIG. 4, while still referring to FIG. 2, the approximating component 212 can generally, using an approximating function 252, approximate at least first breakdown curves 251A representing the first set of mass spectrometry data 250A. Where the second breakdown curves 251B are not yet approximated, and/or approximated spectral breakdown data 254B has not already been acquired, the approximating component 212 also can approximate the second breakdown curves 252B.

An approximating function 252 can comprise, without being limited thereto, a gaussian, log normal, skewed gaussian, voigt, skewed voigt and/or other approximating function 252, with or without outlier detection.

The approximating 450 can comprise employing one or more different approximating functions 252 to generally fit the breakdown curve data 251 to a shape of an approximating function 252. One or more approximating functions 252 can be employed, as illustrated at the 8 different approximating graphs 400A-H of FIG. 4 (illustrated as a function of ion activation energy per ion intensity). At each graph, the shaded area 410 represents the shape of the approximating function 252, and the scatter points 412 represent the breakdown curve data 251.

As illustrated, such as at the graph 400B, one or more of these scatter points 412 can be outliers 412O that would not otherwise have been identified as outliers based on existing frameworks that do not employ such approximating as employed by the one or more embodiments described herein.

Furthermore, use of the approximating 450 can allow for interpolation, filling in, expansion, etc. of data between the scatter points 412, thus providing data at additional ion activation energies at which the mass spectrometry data 250 and/or breakdown curve data 251 was not acquired by the identifying component 210 (e.g., at which the measurement device 249 did not operate spectrometric analysis/fragmentation).

It is noted that the approximating graphs 400 are each generated, by the approximating component 212, for a single fragmentation ion (m/z ion) of the breakdown curve data 251A illustrated at FIG. 3. Thus, the approximating 450 can be performed, by the approximating component 212, for one or more, such as each, of the fragmentation ions (m/z ions) of the breakdown curve data 251A (and/or of the breakdown curve data 251B). It also is noted that the approximating graphs 400 at FIG. 4 each represent only a portion (e.g., a selected range of ion activation energy of the full breakdown curve data 251A for the ion at 81.033491 m/z, for purposes of efficient illustration.

In one or more cases, where two or more approximating functions 252 are employed, the approximating component 212, and/or a user entity having access to the approximating 450 data (such as by use of a computer device communicatively couplable to the non-limiting system 200), can determine a best approximating function 252 to employ for each fragmentation ion. That is, different approximating functions 252 can be employed for different fragmentation ions (e.g., for different breakdown curve data representing different breakdown curve lines).

In one or more embodiments, this determination can be based on best fit. For example, a best approximation function can be determined using a suitable statistical parameter. In one non-limiting case, the function with the minimal area of uncertainty can be identified as the best approximation. The minimal area of uncertainty can be calculated directly by use of a software statistical package. As an example, in Fig. 4, 400D is the best approximation relative to the set of approximating graphs 450.

Based on the approximating 450, as illustrated at FIG. 5, while still referring to FIG. 2, each set of breakdown curve data 251, for each of the breakdown curves 251A, has been approximated (e.g., fit) by the approximating component 212. Resulting is approximated spectral breakdown data 254 (also herein referred to just as spectral breakdown data 254). The spectral breakdown data 254A is illustrated at graph 500 of FIG. 4 as a function of ion activation energy per ion intensity.

Still referring to FIGS. 2 and 5, using the spectral breakdown data 254, the generating component 214 can generate target spectrum data 510 defining a target spectrum 512. In particular, due to the approximating of data between the scatter datapoints 412, target spectrum data 510 at a target ion activation energy 260T can be determined from the spectral breakdown data 254. Put another way, target spectrum data 510 at an ion activation energy that was not employed by the measurement device 249, and/or for which mass spectrometry data 250 and/or breakdown curve data 251 was not acquired, can be obtained in view of the approximating 250.

For example, as illustrated at FIG. 5, a target spectrum 512 can be constructed, by the generating component 214, based on determining, by the generating component 214, of target spectrum data 510 at the target ion activation energy of NCE 94.

This is but one example. Indeed, in use, the generating component 214 can generate target spectrum data 510 for a plurality of data points including, and/or in between, the scatter points 412, to thereby generate/identify a set of comprehensive data that can be employed by the comparing component 216 for the comparison 270. Any suitable range, frequency and/or deviation between individual sets of spectrum data can be employed.

Discussion next turns to a second set of one or more processes that can comprise executing a comparison 270 of sets of spectral breakdown data 254 resulting from the approximating 450 (FIG. 4) of the breakdown curve data 251 (which resulted in the fitted data at FIG. 5).

In one or more embodiments, the second set can comprise the following non-limiting set of steps:

| 1 | func SimilarityAtE(DBC1, BDC2, Energy) |
|---|---|
| 2 | Input: BDC1, BDC2 and Energy |
| 3 | Spectrum1 = BDC1.GetSpectrum(Energy) |
| 4 | Spectrum2 = BDC2.GetSpectrum(Energy) |
| 5 | Similarity = Cos(Spectrum1, Specytrum2) |
| 6 | return Similarity |

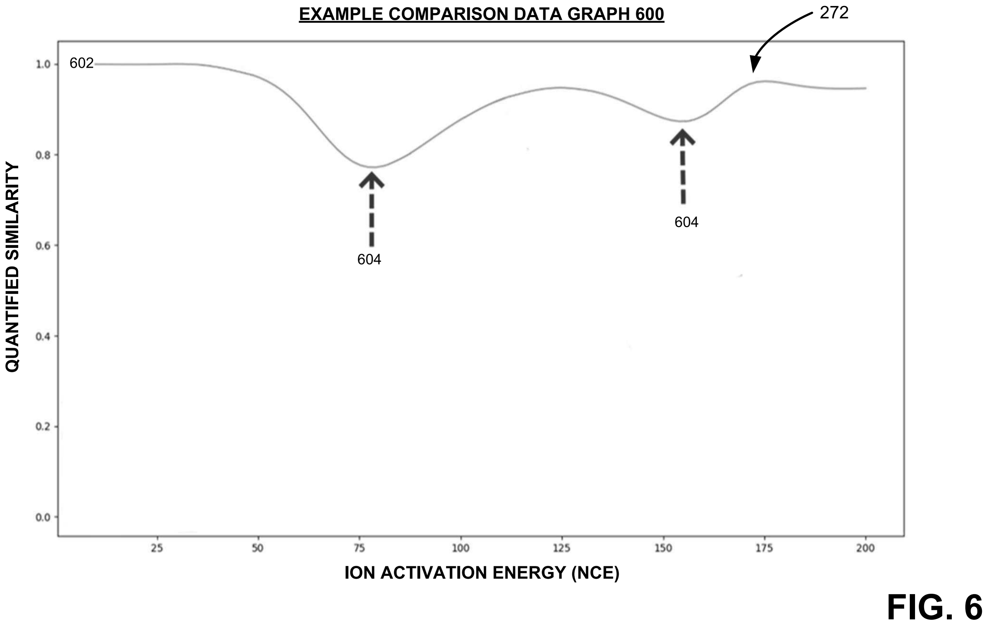

Accordingly, turning next to FIG. 6, and still referring to FIG. 2, the comparing component 216 can execute a comparison 270 of the first set of spectral breakdown data 254A to the second set of spectral breakdown data 254B at least at the target ion activation energy 260T, resulting in a target similarity value 272T defining a similarity between the first set of spectral breakdown data 254A and the second set of spectral breakdown data 254B at the target ion activation energy 260T. Looking to graph 600 of FIG. 6, this comparison can be performed for a plurality of ion activation energies, such as a range of ion activation energies 260R, over which the spectral breakdown data 254A and 254B have been generated based on the approximating 450. Again, any suitable range, frequency and/or deviation between individual sets of spectrum data can be employed.

The comparing component 216 can use any suitable similarity metric, such as, but not limited to, cosine similarity, National Institute of Standards and Technology (NIST) score, etc. As illustrated at FIG. 6, a cosine similarity was employed as the quantified similarity metric for the data corresponding to an individual fragmentation ion compared relative to FIG. 6.

Put another way, as a result of the comparison 270, a comprehensive understanding of local minima and/or local maxima for individual fragmentation ions and/or for whole compounds (e.g., 230A, 230B) can be obtained. For example, graph 600 illustrates comparison data 272 comprising ion activation energy per quantified similarity for a single fragmentation ion 602. As illustrated, a cosine similarity of 1.0 can represent exact similarity, while values less than 1.0 can represent less similarity. A pair of local minima 604 (e.g., local lowest value) are comprised by the comparison data 272 at a pair of different ion activation energies, thus representing the ion activation energies at which the two sets of spectral breakdown data 254A, 254B are most dissimilar. At local minima, similarity is at its lowest and the dissimilarity is at its highest. Conversely, at local maxima, similarity is at its highest and the dissimilarity is at its lowest.

In one example, one of the local minima 604 can be represented by a target similarity value 272T that can be at the target ion activation energy 260T. Such target similarity value 272T could not have been generated using existing frameworks.

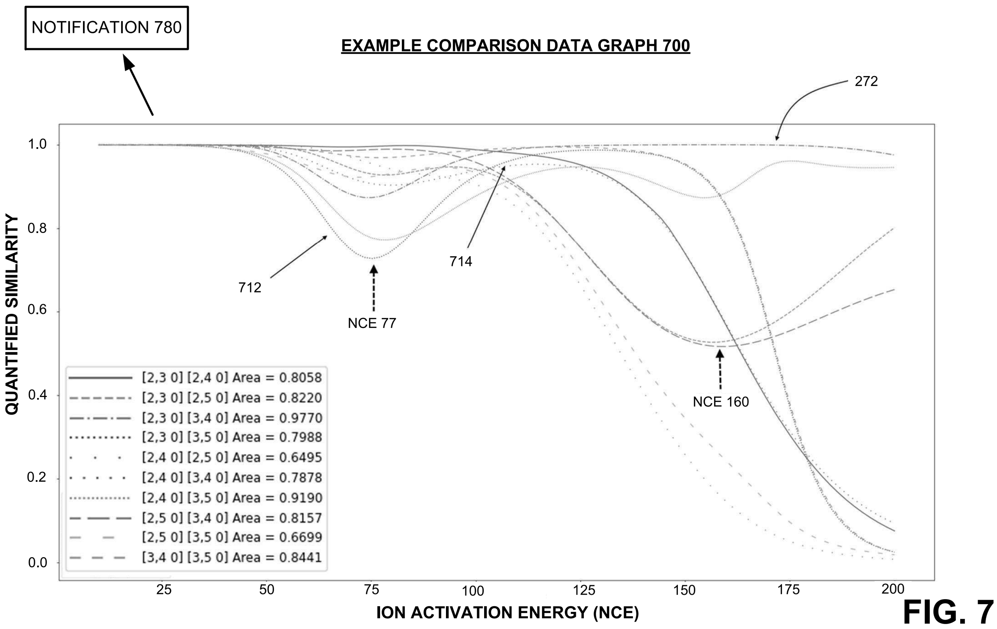

Turning next to FIG. 7, and still referring to FIG. 2, as noted above, the comparison component 216 can generate comparison data 272 for two or more fragmentation ions (e.g., those common between the sets of spectral breakdown data 254A, 254B). That is, graph 700 represents comparison data 272 for a range of ion activation energies 260R for a plurality of fragmentation ions. At graph 700, each separately (e.g., differently) dotted line represents comparison data for a different individual fragmentation ion.

Note that if an ion is present in first compound but not in second, it means that the intensity of this ion in the second compound is zero. In this case, a zero intensity can be assigned to the missing ion in the second compound during the spectra similarity computation. The ions that are not common to both BDCs can be associated with strong informational value and influence the spectra similarity.

In connection therewith, in addition to similarity values for single fragmentation ions, the outputting component 218 also can generate total spectra similarity data 272S representing comparison/similarities of the full sets (or partial sets) of sets spectral breakdown data 254A, 254B. As illustrated at FIG. 7, local minima 712 and/or local maxima 714 of total similarity can be identified by the comparing component 214 using the total spectra similarity data 272S. For example, total similarity can be represented by an area under each curve at graph 700.

In one or more embodiments, total similarity data 272S can be obtained using Equation 1, where E represents the ion activation energy, and resulting in a similarity from range <0,1>, where value 1 means that the two BDCs and representing compounds (e.g., combination of their fragmentation ions) are indistinguishable using MS spectrum.

Equation 1:

$$Totatl\ Similarity = \frac{\int_{E_{min}}^{E_{max}} SimilarityAtE(BDC_1, BDC_2, E)dE}{E_{max} - E_{min}}$$

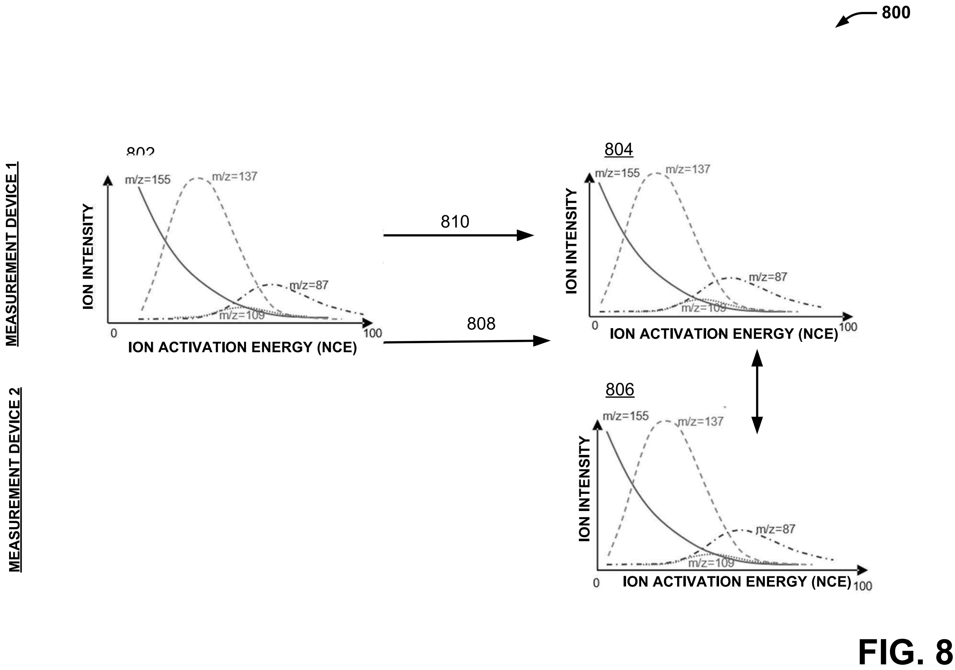

Turning next to FIG. 8, in one or more embodiments, where mass spectrometry data 250, breakdown curve data 251 and/or approximated spectral breakdown data 254 is desired to be compared as generated by a pair of different measurement devices 249 (e.g., measurement device 1 and measurement device 2 at FIG. 8), a normalization of ion activation energies employed by the different measurement devices 249, and subsequent transformation of spectral breakdown data 254 for at least one of the sets of spectral breakdown data 254A, 254B can be performed by the comparing component 216 and/or outputting component 218.

For example, the comparing component 216 can generate, and the outputting component 218 can output, the total spectra similarity data 272S based on a normalization 808 of the range of ion activation energies 260R between a first spectrometry device 249, at which the prior spectrometry measurement of the first compound 230A was performed, and a second spectrometry device (e.g., another measurement device 249), at which spectrometry measurement of the second compound 230B was performed.

Normalization can be performed based on use of a set of three equations (Equations 2, 3 and 4), where $E_1$ represents energy from one device and $E_2$ represents energy from a second device using parameters a and b. Parameters a and b are optimized by employing Equation 3. With these equations, a total similarity can be employed to define transformation of energies between the two different devices 1 and 2. For example, if the transformation has a linear form:

Equation 2: $E_1 = a\ E_2 + b$

Equation 3:

$$\frac{\int_{E_{min}}^{E_{max}} SimilarityAtE(BDC1(E), BDC2(a\ E + b))dE}{E_{max} - E_{min}} = min$$

Equation 4:

$$Totatl\ Similarity = \frac{\int_{E_{min}}^{E_{max}} SimilarityAtE(BDC_1, BDC_2, E)dE}{E_{max} - E_{min}}$$

After normalization of the ion activation energies 260A and/or 260B to one another, the resulting spectral breakdown data 254 can be transformed via a transformation (e.g., a shifting) 810 performed by the comparing component 216 and output by the outputting component 218. For example, as illustrated at FIG. 8, the spectral breakdown data 254A can be transformed based on normalization of the first ion activation energies 260A relative to the second ion activation energies 260B.

Discussion next turns to a third set of one or more processes that can comprise evaluation of comparison data 272 output from the comparison 270.

That is, still referring to FIG. 2, discussion turns to evaluation of (e.g., use of) the comparison data 272 by the evaluating component 220, notifying component 222 and/or graphing component 224.

For example, looking again to FIG. 7, the evaluating component 220 can identify an output ion activation energy (e.g., output ion activation energy 712), being the target ion activation energy 260T or another ion activation energy, from the range of ion activation energies 260R, as corresponding to a maximum difference in quantified similarity (e.g., lowest quantified similarity value of the comparison data 272) between the first set of spectral breakdown data 254A and the second set of spectral breakdown data 254B. For example, similarity 1 means that both sets are identical. Value 0 means that both sets are absolutely different. Searching for the maximum difference involves identifying the minimal value (local minima, e.g., 604).

In response to such identification, the notifying component 222 can generate a notification 280 comprising data requesting re-fragmenting of the first compound at the output ion activation energy. The notification 280 can be communicated and/or otherwise made available to a user entity, such as to a computer device associated with the user entity and communicatively couplable to the non-limiting system 200.

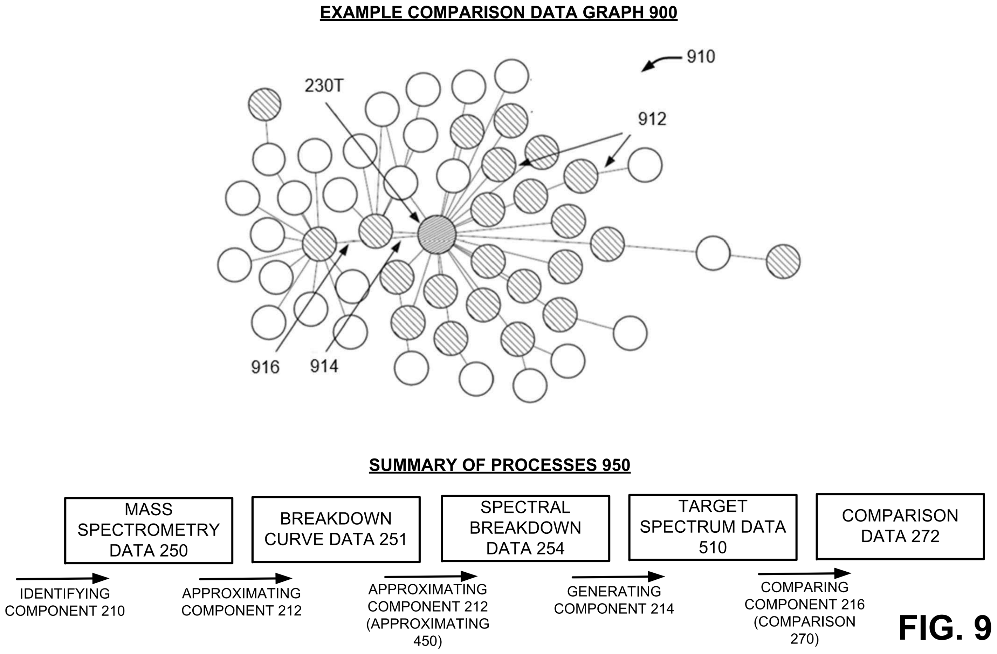

For another example, looking now to FIG. 9, the graphing component 224 can generate graph data 910 defining a node-based graph 900 comprising nodes 912 corresponding to the first compound 230A and the second compound 230B, and edges 914 extending between the nodes 912 and corresponding to a total spectra similarity value 916, of the total spectra similarity data 272S, between the first compound 230A and the second compound 230B.

As a summary of the above-described components and/or functions thereof, a summary of processes 950 is provided at FIG. 9. As illustrated, mass spectrometry data 250 can be identified by the identifying component, with subsequent breakdown curve data 251 being generated by the approximating component 212. As noted above, and in view of this summary being non-limiting, the identifying component 210 can identify the breakdown curve data 251 in one or more embodiments.

The approximating component 212, performing the approximating 450 can generate the spectral breakdown data 254 by using the breakdown curve data 251. The generating component 214 can generate the target spectrum data 510 by using the spectral breakdown data 254. The comparing component 216 can perform the comparison 270 to generate the comparison data 272 based on the target spectrum data 510 and spectral breakdown data 254.

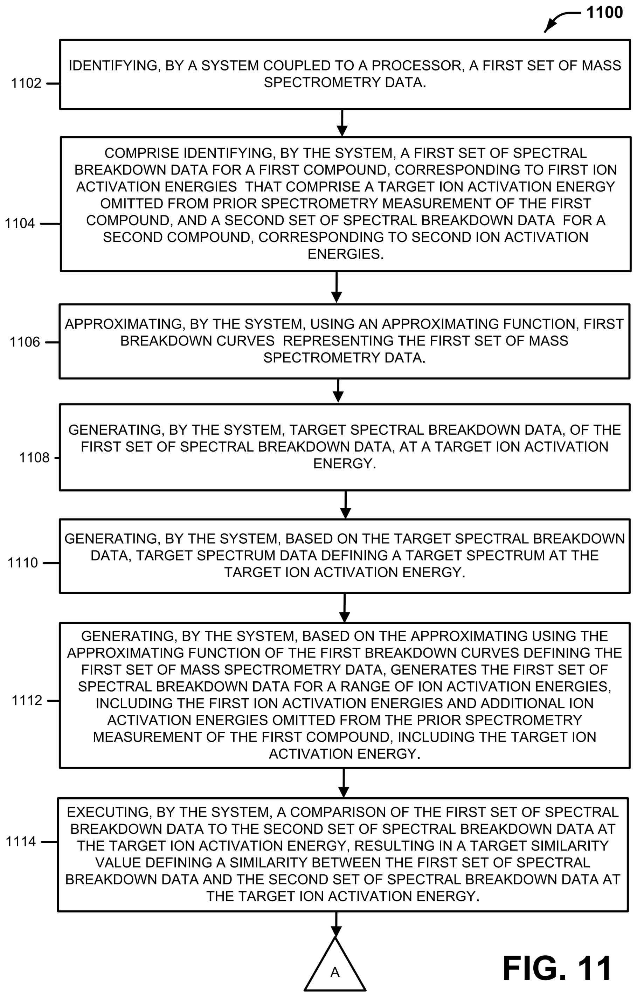

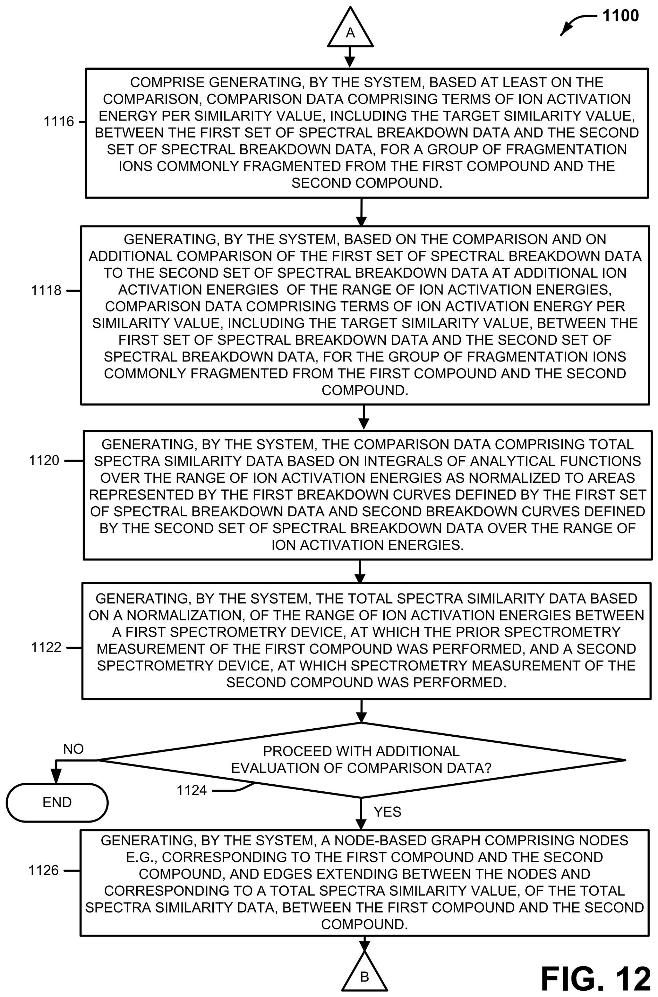

As another summary of the above-described components and/or functions thereof, referring next to FIGS. 11 and 12, illustrated is a flow diagram of an example, non-limiting method 1100 that can facilitate a process for measurement device output comparison and/or evaluation, in accordance with one or more example embodiments described herein, such as the non-limiting system 200 of FIG. 2. While the non-limiting method 1100 is described relative to the non-limiting system 200 of FIG. 2, the non-limiting method 1100 can be applicable also to other systems described herein, such as the non-limiting system 100 of FIG. 1. Repetitive description of like elements and/or processes employed in respective embodiments is omitted for sake of brevity.

At 1102, the non-limiting method 1100 can comprise identifying, by a system (e.g., identifying component 210) coupled to a processor (e.g., processor 206), a first set of mass spectrometry data (e.g., first set of mass spectrometry data 250A).

At 1104, the non-limiting method 1100 can comprise identifying, by the system (e.g., identifying component 210), a first set of spectral breakdown data for a first compound (e.g., first compound 230A), corresponding to first ion activation energies (e.g., first ion activation energies 260A) that comprise a target ion activation energy (e.g., target ion activation energy, 260T) omitted from prior spectrometry measurement of the first compound, and a second set of spectral breakdown data (e.g., second set of spectral breakdown data 254B) for a second compound (e.g., second compound 230B), corresponding to second ion activation energies (e.g., second ion activation energies 260B).

As noted above, the first compound 230A can be same or different from the second compound 230B, or vice versa. As also noted above, the first ion activation energies 260A can be the same or different from the second ion activation energies 260B, or vice versa.

At 1106, the non-limiting method 1100 can comprise approximating, by the system (e.g., approximating component 212), using an approximating function (e.g., approximating function 252), first breakdown curves (e.g., first breakdown curves 251A) representing the first set of mass spectrometry data (first set of mass spectrometry data 250A).

At 1108, the non-limiting method 1100 can comprise generating, by the system (e.g., generating component 214), target spectral breakdown data (e.g., target spectral breakdown data 254T), of the first set of spectral breakdown data (e.g., first set of spectral breakdown data 254A), at a target ion activation energy (e.g., target ion activation energy 260T).

At 1110, the non-limiting method 1100 can comprise generating, by the system, (e.g., generating component 214), based on the target spectral breakdown data, target spectrum data (e.g., target spectrum data 510) defining a target spectrum (e.g., target spectrum 512) at the target ion activation energy (e.g., target ion activation energy 260T).

At 1112, the non-limiting method 1100 can comprise generating, by the system (e.g., generating component 214), based on the approximating using the approximating function of the first breakdown curves (e.g., first breakdown curves 251A) defining the first set of mass spectrometry data (e.g., first set of mass spectrometry data 250A), generates the first set of spectral breakdown data (e.g., first set of spectral breakdown data 254A) for a range of ion activation energies (e.g., range of ion activation energies 260R), including the first ion activation energies (e.g., first ion activation energies 260A) and additional ion activation energies (e.g., additional ion activation energies 260X) omitted from the prior spectrometry measurement of the first compound, including the target ion activation energy (e.g., target ion activation energy 260T).

At 1114, the non-limiting method 1100 can comprise executing, by the system (e.g., comparing component 216), a comparison (e.g., comparison 270) of the first set of spectral breakdown data (e.g., first set of spectral breakdown data 254A) to the second set of spectral breakdown data (e.g., second set of spectral breakdown data 254B) at the target ion activation energy (e.g., target ion activation energy 260T), resulting in a target similarity value (e.g., target similarity value 272T) defining a similarity between the first set of spectral breakdown data and the second set of spectral breakdown data

at the target ion activation energy.

At 1116, the non-limiting method 1100 can comprise generating, by the system (e.g., outputting component 218), based at least on the comparison, comparison data (e.g., comparison data 272) comprising terms of ion activation energy per similarity value (e.g., data defining two lines at graph 500 at FIG. 5), including the target similarity value, between the first set of spectral breakdown data and the second set of spectral breakdown data, for a group of fragmentation ions (e.g., fragmentation ions 232) commonly fragmented from the first compound and the second compound.

At 1118, the non-limiting method 1100 can comprise generating, by the system (e.g., outputting component 218), based on the comparison (e.g., comparison 270) and on additional comparison (e.g., comparison 270X) of the first set of spectral breakdown data to the second set of spectral breakdown data at additional ion activation energies (e.g., additional ion activation energies 260X) of the range of ion activation energies, comparison data (e.g., comparison data 272) comprising terms of ion activation energy per similarity value (e.g., data defining plural lines at graph 500 at FIG. 5), including the target similarity value (e.g., target similarity value 272T), between the first set of spectral breakdown data (e.g., first set of spectral breakdown data 254A) and the second set of spectral breakdown data (e.g., second set of spectral breakdown data 254B), for the group of fragmentation ions (e.g., fragmentation ions 232) commonly fragmented from the first compound and the second compound.

At 1120, the non-limiting method 1100 can comprise generating, by the system (e.g., outputting component 218), the comparison data (e.g., comparison data 272) comprising total spectra similarity data (e.g., total spectra similarity data 272S) based on integrals of analytical functions over the range of ion activation energies as normalized to areas represented by the first breakdown curves (e.g., first breakdown curves 251A) defined by the first set of spectral breakdown data and second breakdown curves (e.g., second breakdown curves 251B) defined by the second set of spectral breakdown data over the range of ion activation energies.

At 1122, the non-limiting method 1100 can comprise generating, by the system (e.g., outputting component 218), the total spectra similarity data (e.g., total spectra similarity data 272) based on a normalization (e.g., normalization 808), of the range of ion activation energies between a first spectrometry device (e.g., measurement device 249), at which the prior spectrometry measurement of the first compound was performed, and a second spectrometry device (e.g., another measurement device 249), at which spectrometry measurement of the second compound was performed.

At 1124, the non-limiting method 1100 can comprise determining, by the system (e.g., evaluating component 220), whether to proceed with additional evaluation of the comparison data (e.g., comparison data 272) resulting from the outputting. If not, the non-limiting method 1100 can proceed to end. If yes, the non-limiting method can proceed to step 1122.

At 1126, the non-limiting method 1100 can comprise generating, by the system (e.g., graphing component 224), a node-based graph (e.g., graph 900) comprising nodes (e.g., nodes 912) corresponding to the first compound and the second compound, and edges (e.g., edges 914) extending between the nodes and corresponding to a total spectra similarity value (e.g., total spectra similarity value 916), of the total spectra similarity data (e.g., total spectra similarity data 272S), between the first compound and the second compound.

At 1128, the non-limiting method 1100 can comprise identifying, by the system (e.g., evaluating component 220), an output ion activation energy (e.g., output ion activation energy 712), being the target ion activation energy or another ion activation energy, from the range of ion activation energies, as corresponding to a maximum difference in quantified similarity between the first set of spectral breakdown data and the second set of spectral breakdown data.

At 1130, the non-limiting method 1100 can comprise generating, by the system (e.g., notifying component 222), in response to an identification of the output ion activation energy, a notification (e.g., notification 280) requesting re-fragmenting of the first compound at the output ion activation energy.

Additional Summary

For simplicity of explanation, the computer-implemented and non-computer-implemented methodologies provided herein are depicted and/or described as a series of acts. It is to be understood that the subject innovation is not limited by the acts illustrated and/or by the order of acts, for example acts can occur in one or more orders and/or concurrently, and with other acts not presented and described herein. Furthermore, not all illustrated acts can be utilized to implement the computer-implemented and non-computer-implemented methodologies in accordance with the described subject matter. In addition, the computer-implemented and non-computer-implemented methodologies could alternatively be represented as a series of interrelated states via a state diagram or events. Additionally, the computer-implemented methodologies described hereinafter and throughout this specification are capable of being stored on an article of manufacture for transporting and transferring the computer-implemented methodologies to computers. The term article of manufacture, as used herein, is intended to encompass a computer program accessible from any computer-readable device or storage media.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

In summary, one or more systems, computer program products and/or computer-implemented methods provided herein and/or described herein relate to evaluating similarity between spectral datasets. A system can comprise a memory 104, 204 that stores, and a processor 106, 206 that executes, computer executable components. The computer executable components can comprise an identifying component 110, 210 that identifies a first set of spectral breakdown data 154A, 254A for a first compound 130A, 230A, corresponding to first ion activation energies 160A, 260A that comprise a target ion activation energy 160T, 260T omitted from prior spectrometry measurement of the first compound 130A, 230A, wherein the identifying component 110, 210 further identifies a second set of spectral breakdown data 154B, 254B for a second compound 130B, 230B, corresponding to second ion activation energies 160B, 260B, and a comparing component 116, 216 that executes a comparison of the first set of spectral breakdown data 154A, 254A to the second set of spectral breakdown data 154B, 254B at the target ion activation energy 160T, 260T, resulting in a target similarity value 172T, 272T defining a similarity between the first set of spectral breakdown data 154A, 254A and the second set of spectral breakdown data 154B, 254B at the target ion activation energy 160T, 260T.

The one or more example embodiments disclosed herein can be applied on a plug-and-play basis to various architectures of one or more measurement devices, a same measurement device using plural exchangeable components, etc. for calibration, normalization and/or comparison of output data relative to unknown, known and/or standard data. The frameworks described herein can be performed in a time efficient and at least partially automatic manner, thereby increasing device use time and/or reducing user entity interaction for pre-experiment and/or post-experiment processes..

Accordingly, the one or more example embodiments described herein can be implemented within, in connection with and/or coupled to a scientific measurement device.

Indeed, in view of the one or more example embodiments described herein, a practical application of the one or more systems, computer-implemented methods and/or computer program products described herein can be an ability to interpolate spectral data corresponding to ion activation energies not specifically employed during spectrometric operations. This can be accomplished by employing approximating functions to breakdown data corresponding to one or more fragment ions fragmented from a compound at two or more other ion activation energies. In this way, comparison between breakdown data for different compounds, different devices, same compound but different devices, etc. can be made over a range of ion activation energies without directly obtaining spectral data at all ion activation energies of the range of ion activation energies. As a result, the one or more embodiments described herein can reduce a number of fragmentation runs to be performed at a measurement device to obtain initial mass spectrometry data from which the breakdown data is generated.

As compared to existing frameworks that cannot provide this ability, the one or more example embodiments described herein can provide a new result that was previously unavailable. That is, based on the use of approximated breakdown data, a more comprehensive understanding of the breakdown data, as compared to existing frameworks over a range of ion activation energies can be obtained. This can allow for identification of local minima or local maxima of quantified similarity between and/or among two or more sets of breakdown data, with at least one of the sets comprising approximated breakdown data. Optionally, all sets of breakdown data being compared can comprise approximated breakdown data.

These are useful and practical applications of computers, thus providing enhanced (e.g., improved and/or optimized) spectral data comparison and/or breakdown data comparison corresponding to initial spectral data. Overall, such computerized tools can constitute a concrete and tangible technical improvement in the fields of material analysis, and more particularly in analysis of scientific measurement device output, such as including, but not limited to, the field of spectrometry.

Furthermore, one or more example embodiments described herein can be employed in a real-world system based on the disclosed teachings. For example, a local minima and/or local maxima of quantified similarity between spectral data sets can be determined, corresponding to a one or more ion activation energies, for a specified measurement device, for plural specified measurement devices, for a same compound, for different compounds, etc., without being limited thereto. Based on the quantified similarity data generated, fragmentation at one or more ion activation energies can be reevaluated and/or re-performed, a databased can be generated and/or updated using total similarity values (e.g., quantified similarity over a range of ion activation energies) to link compounds, molecules and/or ions, and/or differences between data output from plural measurement devices can be employed for comparison, error-correcting and/or calibration purposes. These can be useful processes for varying industries employing material analysis, product manufacturing, quality control and/or the like. The embodiments disclosed herein thus can provide improvements to scientific instrument technology (e.g., improvements in the computer technology supporting such scientific instruments, among other improvements).

Moreover, the one or more example embodiments described herein can achieve a level of scale of operation. For example, spectral data corresponding to two or more compounds can be evaluated at least partially in parallel with one another relative to same and/or different compounds, measurement devices, time periods and/or fragment ions.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

One or more example embodiments described herein can be, in one or more example embodiments, inherently and/or inextricably tied to computer technology and cannot be implemented outside of a computing environment. For example, one or more processes performed by one or more example embodiments described herein can more efficiently, and even more feasibly, provide program and/or program instruction execution, such as relative to measurement device output comparison (e.g., measurement device use for material analysis), as compared to existing systems and/or techniques using molecular network generation and/or visualization. Systems, computer-implemented methods and/or computer program products providing performance of these processes are of great utility in the fields of material analysis and cannot be equally practicably implemented in a sensible way outside of a computing environment.

One or more example embodiments described herein can employ hardware and/or software to solve problems that are highly technical, that are not abstract, and that cannot be performed as a set of mental acts by a human. For example, a human, or even thousands of humans, cannot efficiently, accurately and/or effectively analyze computer data/metadata (e.g., spectral data and/or breakdown data) defining fragmentation intensity, mass-to-charge ratio, retention time, etc. of compounds analyzed at one or more measurement devices, and/or generate a digital display visual of quantified similarities between spectral datasets compared, as the one or more example embodiments described herein can provide this process. Moreover, neither can the human mind nor a human with pen and paper conduct one or more of these processes, as conducted by one or more example embodiments described herein.

In one or more example embodiments, one or more of the processes described herein can be performed by one or more specialized computers (e.g., a specialized processing unit, a specialized classical computer, a specialized quantum computer, a specialized hybrid classical/quantum system and/or another type of specialized computer) to execute defined tasks related to the one or more technologies describe above. One or more example embodiments described herein and/or components thereof can be employed to solve new problems that arise through advancements in technologies mentioned above, employment of quantum computing systems, cloud computing systems, computer architecture and/or another technology.

One or more example embodiments described herein can be fully operational towards performing one or more other functions (e.g., fully powered on, fully executed and/or another function) while also performing one or more of the one or more operations described herein.

To provide additional summary, a listing of embodiments and features thereof is next provided.

A system, comprising: a memory that stores computer executable components; and a processor that executes the computer executable components stored in the memory, wherein the computer executable components comprise: an identifying component that identifies a first set of spectral breakdown data for a first compound, corresponding to first ion activation energies that comprise a target ion activation energy omitted from prior spectrometry measurement of the first compound,
wherein the identifying component further identifies a second set of spectral breakdown data for a second compound, corresponding to second ion activation energies; and a comparing component that executes a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

The system of the preceding paragraph, wherein the computer executable components further comprise: an approximating component that approximates, using an approximating function, first breakdown curves representing a first set of mass spectrometry data.

The system of any preceding paragraph, herein the computer executable components further comprise: a generating component that generates target spectral breakdown data, of the first set of spectral breakdown data, at the target ion activation energy, and wherein the generating component, based on the target spectral breakdown data, generates target spectrum data defining a target spectrum at the target ion activation energy.

The system of any preceding paragraph, wherein the computer executable components further comprise: a generating component that, based on an approximating using an approximating function of first breakdown curves defining a first set of mass spectrometry data, generates the first set of spectral breakdown data for a range of ion activation energies, including the first ion activation energies and additional ion activation energies omitted from the prior spectrometry measurement of the first compound, including the target ion activation energy.

The system of any preceding paragraph, wherein the computer executable components further comprise: an outputting component that, based at least on the comparison, generates comparison data comprising terms of ion activation energy per similarity value, including the target similarity value, between the first set of spectral breakdown data and the second set of spectral breakdown data, for a group of fragmentation ions commonly fragmented from the first compound and the second compound.

The system of any preceding paragraph, wherein the computer executable components further comprise: an evaluating component that identifies an output ion activation energy, being the target ion activation energy or another ion activation energy, from the range of ion activation energies, as corresponding to a maximum difference in quantified similarity between the first set of spectral breakdown data and the second set of spectral breakdown data; and a notifying component that, in response to an identification of the output ion activation energy, generates a notification requesting re-fragmenting of the first compound at the output ion activation energy.

The system of any preceding paragraph, wherein the computer executable components further comprise: an outputting component that, based on the comparison and on additional comparison of the first set of spectral breakdown data to the second set of spectral breakdown data at additional ion activation energies of the range of ion activation energies, generates comparison data comprising terms of ion activation energy per similarity value, including the target similarity value, between the first set of spectral breakdown data and the second set of spectral breakdown data, for the group of fragmentation ions commonly fragmented from the first compound and the second compound, wherein the comparison data comprises total spectra similarity data based on integrals of an analytical functions over the range of ion activation energies as normalized to areas represented by the first breakdown curves defined by the first set of spectral breakdown data and second breakdown curves defined by the second set of spectral breakdown data over the range of ion activation energies.

The system of any preceding paragraph, wherein the computer executable components further comprise: a graphing component that generates a node-based graph comprising nodes corresponding to the first compound and the second compound, and edges extending between the nodes and corresponding to a total spectra similarity value, of the total spectra similarity data, between the first compound and the second compound.

The system of any preceding paragraph, wherein the total spectra similarity data is further based on a normalization of the range of ion activation energies between a first spectrometry device, at which the prior spectrometry measurement of the first compound was performed, and a second spectrometry device, at which spectrometry measurement of the second compound was performed.

A computer-implemented method, comprising: identifying, by a system operatively coupled to a processor, a first set of spectral breakdown data for a first compound, corresponding to first ion activation energies that comprise a target ion activation energy omitted from prior spectrometry measurement of the first compound; identifying, by the system, a second set of spectral breakdown data for a second compound, corresponding to second ion activation energies; and executing, by the system, a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

The computer-implemented method of the preceding paragraph, further comprising: approximating, by the system, using an approximating function, first breakdown curves representing a first set of mass spectrometry data. The computer-implemented method of any preceding paragraph, further comprising: generating, by the system, target spectral breakdown data, of the first set of spectral breakdown data, at the target ion activation energy; and generating, by the system, based on the target spectral breakdown data, target spectrum data defining a target spectrum at the target ion activation energy.

The computer-implemented method of any preceding paragraph, further comprising: generating, by the system, based on an approximating using an approximating function of first breakdown curves defining a first set of mass spectrometry data, the first set of spectral breakdown data for a range of ion activation energies, including the first ion activation energies and additional ion activation energies omitted from the prior spectrometry measurement of the first compound, including the target ion activation energy.

The computer-implemented method of any preceding paragraph, further comprising: generating, by the system, based at least on the comparison, comparison data comprising terms of ion activation energy per similarity value, including the target similarity value, between the first set of spectral breakdown data and the second set of spectral breakdown data, for a group of fragmentation ions commonly fragmented from the first compound and the second compound.

The computer-implemented method of any preceding paragraph, further comprising: identifying, by the system, an output ion activation energy, being the target ion activation energy or another ion activation energy, from the range of ion activation energies, as corresponding to a maximum difference in quantified similarity between the first set of spectral breakdown data and the second set of spectral breakdown data; and generating, by the system, in response to an identification of the output ion activation energy, a notification requesting re-fragmenting of the first compound at the output ion activation energy.

A computer program product facilitating a process for evaluating similarity between spectral datasets, the computer program product comprising a computer readable storage medium having program instructions embodied therewith, and the program instructions executable by a processor to cause the processor to: identify, by the processor, a first set of spectral breakdown data for a first compound, corresponding to first ion activation energies that comprise a target ion activation energy omitted from prior spectrometry measurement of the first compound; identify, by the processor, a second set of spectral breakdown data for a second compound, corresponding to second ion activation energies; and execute, by the processor, a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

The computer program product of the preceding paragraph, wherein the program instructions are further executable by the processor to cause the processor to: approximate, by the processor, using an approximating function, first breakdown curves representing a first set of mass spectrometry data.

The computer program product of any preceding paragraph, wherein the program instructions are further executable by the processor to cause the processor to: generate, by the processor, target spectral breakdown data, of the first set of spectral breakdown data, at the target ion activation energy; and generate, by the processor, based on the target spectral breakdown data, target spectrum data defining a target spectrum at the target ion activation energy.

The computer program product of any preceding paragraph, wherein the program instructions are further executable by the processor to cause the processor to: generate, by the processor, based on an approximating using an approximating function of first breakdown curves defining a first set of mass spectrometry data, the first set of spectral breakdown data for a range of ion activation energies, including the first ion activation energies and additional ion activation energies omitted from the prior spectrometry measurement of the first compound, including the target ion activation energy; and generate, by the processor, based at least on the comparison, comparison data comprising terms of ion activation energy per similarity value, including the target similarity value, between the first set of spectral breakdown data and the second set of spectral breakdown data, for a group of fragmentation ions commonly fragmented from the first compound and the second compound.

The computer program product of any preceding paragraph, wherein the program instructions are further executable by the processor to cause the processor to: generate, by the processor, based on an approximating of a first set of mass spectrometry data using an approximating function, the first set of spectral breakdown data for a range of ion activation energies, including the first ion activation energies and additional ion activation energies omitted from the prior spectrometry measurement of the first compound, including the target ion activation energy; identify, by the processor, an output ion activation energy, being the target ion activation energy or another ion activation energy, from the range of ion activation energies, as corresponding to a maximum difference in quantified similarity between the first set of spectral breakdown data and the second set of spectral breakdown data; and generate, by the processor, in response to an identification of the output ion activation energy, a notification requesting re-fragmenting of the first compound at the output ion activation energy.

Example Operating Environment

FIG. 14 is a schematic block diagram of an operating environment 1400 with which the described subject matter can interact. The operating environment 1400 comprises one or more remote component(s) 1410. The remote component(s) 1410 can be hardware and/or software (e.g., threads, processes, computing devices). In one or more example embodiments, remote component(s) 1410 can be a distributed computer system, connected to a local automatic scaling component and/or programs that use the resources of a distributed computer system, via communication framework 1440. Communication framework 1440 can comprise wired network devices, wireless network devices, mobile devices, wearable devices, radio access network devices, gateway devices, femtocell devices, servers, etc.

The operating environment 1400 also comprises one or more local component(s) 1420. The local component(s) 1420 can be hardware and/or software (e.g., threads, processes, computing devices). In one or more example embodiments, local component(s) 1420 can comprise an automatic scaling component and/or programs that communicate/use the remote resources 1410 and 1420, etc., connected to a remotely located distributed computing system via communication framework 1440.

One possible communication between a remote component(s) 1410 and a local component(s) 1420 can be in the form of a data packet adapted to be transmitted between two or more computer processes. Another possible communication between a remote component(s) 1410 and a local component(s) 1420 can be in the form of circuit-switched data adapted to be transmitted between two or more computer processes in radio time slots. The operating environment 1400 comprises a communication framework 1440 that can be employed to facilitate communications between the remote component(s) 1410 and the local component(s) 1420, and can comprise an air interface, e.g., interface of a UMTS network, via an LTE network, etc. Remote component(s) 1410 can be operably connected to one or more remote data store(s) 1450, such as a hard drive, solid state drive, subscriber identity module (SIM) card, electronic SIM (eSIM), device memory, etc., that can be employed to store information on the remote component(s) 1410 side of communication framework 1440. Similarly, local component(s) 1420 can be operably connected to one or more local data store(s) 1430, that can be employed to store information on the local component(s) 1420 side of communication framework 1440.

Example Computing Environment

In order to provide additional context for various embodiments described herein, FIG. 15 and the following discussion are intended to provide a brief, general description of a suitable computing environment 1500 in which the various embodiments of the embodiment described herein can be implemented. While the embodiments have been described above in the general context of computer-executable instructions that can run on one or more computers, those skilled in the art will recognize that the embodiments can be also implemented in combination with other program modules and/or as a combination of hardware and software.

Generally, program modules include routines, programs, components, data structures, etc., that perform tasks or implement abstract data types. Moreover, the methods can be practiced with other computer system configurations, including single-processor or multiprocessor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can also be practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, and/or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data, or unstructured data.

Computer-readable storage media can include, but are not limited to, random access memory (RAM), read only memory (ROM), electrically erasable programmable read only memory (EEPROM), flash memory or other memory technology, compact disk read only memory (CD ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible and/or non-transitory media which can be used to store desired information. In this regard, the terms "tangible" or "non-transitory" herein as applied to storage, memory, or computer-readable media, exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries, or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

Referring still to FIG. 15, the example computing environment 1500 which can implement one or more example embodiments described herein includes a computer 1502, the computer 1502 including a processing unit 1504, a system memory 1506 and a system bus 1508. The system bus 1508 couples system components including, but not limited to, the system memory 1506 to the processing unit 1504. The processing unit 1504 can be any of various commercially available processors. Dual microprocessors and other multi processor architectures can also be employed as the processing unit 1504.

The system bus 1508 can be any of several types of bus structure that can further interconnect to a memory bus (with or without a memory controller), a peripheral bus, and a local bus using any of a variety of commercially available bus architectures. The system memory 1506 includes ROM 1510 and RAM 1512. A basic input/output system (BIOS) can be stored in a non-volatile memory such as ROM, erasable programmable read only memory (EPROM), EEPROM, which BIOS contains the basic routines that help to transfer information between elements within the computer 1502, such as during startup. The RAM 1512 can also include a high-speed RAM such as static RAM for caching data.

The computer 1502 further includes an internal hard disk drive (HDD) 1514 (e.g., EIDE, SATA), and can include one or more external storage devices 1516 (e.g., a magnetic floppy disk drive (FDD) 1516, a memory stick or flash drive reader, a memory card reader, etc.). While the internal HDD 1514 is illustrated as located within the computer 1502, the internal HDD 1514 can also be configured for external use in a suitable chassis (not shown). Additionally, while not shown in computing environment 1500, a solid-state drive (SSD) could be used in addition to, or in place of, an HDD 1514.

Other internal or external storage can include at least one other storage device 1520 with storage media 1522 (e.g., a solid-state storage device, a nonvolatile memory device, and/or an optical disk drive that can read or write from removable media such as a CD-ROM disc, a DVD, a BD, etc.). The external storage 1516 can be facilitated by a network virtual machine. The HDD 1514, external storage device 1516 and storage device (e.g., drive) 1520 can be connected to the system bus 1508 by an HDD interface 1524, an external storage interface 1526 and a drive interface 1528, respectively.

The drives and their associated computer-readable storage media provide nonvolatile storage of data, data structures, computer-executable instructions, and so forth. For the computer 1502, the drives and storage media accommodate the storage of any data in a suitable digital format. Although the description of computer-readable storage media above refers to respective types of storage devices, other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1512, including an operating system 1530, one or more application programs 1532, other program modules 1534 and program data 1536. All or portions of the operating system, applications, modules, and/or data can also be cached in the RAM 1512. The systems and methods described herein can be implemented utilizing various commercially available operating systems or combinations of operating systems.

Computer 1502 can optionally comprise emulation technologies. For example, a hypervisor (not shown) or other intermediary can emulate a hardware environment for operating system 1530, and the emulated hardware can optionally be different from the hardware illustrated in FIG. 15. In such an embodiment, operating system 1530 can comprise one virtual machine (VM) of multiple VMs hosted at computer 1502. Furthermore, operating system 1530 can provide runtime environments, such as the Java runtime environment or the .NET framework, for applications 1532. Runtime environments are consistent execution environments that allow applications 1532 to run on any operating system that includes the runtime environment. Similarly, operating system 1530 can support containers, and applications 1532 can be in the form of containers, which are lightweight, standalone, executable packages of software that include, e.g., code, runtime, system tools, system libraries and settings for an application.

Further, computer 1502 can be enabled with a security module, such as a trusted processing module (TPM). For instance, with a TPM, boot components hash next in time boot components and wait for a match of results to secured values, before loading a next boot component. This process can take place at any layer in the code execution stack of computer 1502, e.g., applied at the application execution level or at the operating system (OS) kernel level, thereby enabling security at any level of code execution.

A user entity can enter commands and information into the computer 1502 through one or more wired/wireless input devices, e.g., a keyboard 1538, a touch screen 1540, and a pointing device, such as a mouse 1542. Other input devices (not shown) can include a microphone, an infrared (IR) remote control, a radio frequency (RF) remote control, or other remote control, a joystick, a virtual reality controller and/or virtual reality headset, a game pad, a stylus pen, an image input device, e.g., camera, a gesture sensor input device, a vision movement sensor input device, an emotion or facial detection device, a biometric input device, e.g., fingerprint or iris scanner, or the like. These and other input devices are often connected to the processing unit 1504 through an input device interface 1544 that can be coupled to the system bus 1508, but can be connected by other interfaces, such as a parallel port, an IEEE 1394 serial port, a game port, a USB port, an IR interface, a BLUETOOTH® interface, etc.

A monitor 1546 or other type of display device can also be connected to the system bus 1508 via an interface, such as a video adapter 1548. In addition to the monitor 1546, a computer typically includes other peripheral output devices (not shown), such as speakers, printers, etc.

The computer 1502 can operate in a networked environment using logical connections via wired and/or wireless communications to one or more remote computers, such as a remote computer 1550. The remote computer 1550 can be a workstation, a server computer, a router, a personal computer, portable computer, microprocessor-based entertainment appliance, a peer device or other common network node, and typically includes many or all of the elements described relative to the computer 1502, although, for purposes of brevity, only a memory/storage device 1552 is illustrated. The logical connections depicted include wired/wireless connectivity to a local area network (LAN) 1554 and/or larger networks, e.g., a wide area network (WAN) 1556. Such LAN and WAN networking environments are commonplace in offices and companies, and facilitate enterprise-wide computer networks, such as intranets, all of which can connect to a global communications network, e.g., the Internet.

When used in a LAN networking environment, the computer 1502 can be connected to the local network 1554 through a wired and/or wireless communication network interface or adapter 1558. The adapter 1558 can facilitate wired or wireless communication to the LAN 1554, which can also include a wireless access point (AP) disposed thereon for communicating with the adapter 1558 in a wireless mode.

When used in a WAN networking environment, the computer 1502 can include a modem 1560 or can be connected to a communications server on the WAN 1556 via other means for establishing communications over the WAN 1556, such as by way of the Internet. The modem 1560, which can be internal or external and a wired or wireless device, can be connected to the system bus 1508 via the input device interface 1544. In a networked environment, program modules depicted relative to the computer 1502 or portions thereof, can be stored in the remote memory/storage device 1552. The network connections shown are example and other means of establishing a communications link between the computers can be used.

When used in either a LAN or WAN networking environment, the computer 1502 can access cloud storage systems or other network-based storage systems in addition to, or in place of, external storage devices 1516 as described above. Generally, a connection between the computer 1502 and a cloud storage system can be established over a LAN 1554 or WAN 1556 e.g., by the adapter 1558 or modem 1560, respectively. Upon connecting the computer 1502 to an associated cloud storage system, the external storage interface 1526 can, with the aid of the adapter 1558 and/or modem 1560, manage storage provided by the cloud storage system as it would other types of external storage. For instance, the external storage interface 1526 can be configured to provide access to cloud storage sources as if those sources were physically connected to the computer 1502.

The computer 1502 can be operable to communicate with any wireless devices or entities operatively disposed in wireless communication, e.g., a printer, scanner, desktop and/or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH® wireless technologies. Thus, the communication can be a defined structure as with an existing network or simply an ad hoc communication between at least two devices.

Additional Information

The embodiments described herein can be directed to one or more of a system, a method, an apparatus and/or a computer program product at any possible technical detail level of integration. The computer program product can include a computer readable storage medium (or media) having computer readable program instructions thereon for causing a processor to carry out aspects of the one or more example embodiments described herein. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a superconducting storage device and/or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon and/or any suitable combination of the foregoing. A computer readable storage medium, as used herein, is not to be construed as being transitory signals per se, such as radio waves and/or other freely propagating electromagnetic waves, electromagnetic waves propagating through a waveguide and/or other transmission media (e.g., light pulses passing through a fiber-optic cable), and/or electrical signals transmitted through a wire.

Computer readable program instructions described herein can be downloaded to respective computing/processing devices from a computer readable storage medium and/or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of the one or more example embodiments described herein can be assembler instructions, instruction-set-architecture (ISA) instructions, machine instructions, machine dependent instructions, microcode, firmware instructions, state-setting data, configuration data for integrated circuitry, and/or source code and/or object code written in any combination of one or more programming languages, including an object oriented programming language such as Smalltalk, C++ or the like, and/or procedural programming languages, such as the "C" programming language and/or similar programming languages. The computer readable program instructions can execute entirely on a computer, partly on a computer, as a stand-alone software package, partly on a computer and/or partly on a remote computer or entirely on the remote computer and/or server. In the latter scenario, the remote computer can be connected to a computer through any type of network, including a local area network (LAN) and/or a wide area network (WAN), and/or the connection can be made to an external computer (for example, through the Internet using an Internet Service Provider). In one or more example embodiments, electronic circuitry including, for example, programmable logic circuitry, field-programmable gate arrays (FPGA) and/or programmable logic arrays (PLA) can execute the computer readable program instructions by utilizing state information of the computer readable program instructions to personalize the electronic circuitry, in order to perform aspects of the one or more example embodiments described herein.

Aspects of the one or more example embodiments described herein are described with reference to flowchart illustrations and/or block diagrams of methods, apparatus (systems), and computer program products according to one or more example embodiments described herein. It will be understood that each block of the flowchart illustrations and/or block diagrams, and combinations of blocks in the flowchart illustrations and/or block diagrams, can be implemented by computer readable program instructions. These computer readable program instructions can be provided to a processor of a general-purpose computer, special purpose computer and/or other programmable data processing apparatus to produce a machine, such that the instructions, which execute via the processor of the computer or other programmable data processing apparatus, can create means for implementing the functions/acts specified in the flowchart and/or block diagram block or blocks. These computer readable program instructions can also be stored in a computer readable storage medium that can direct a computer, a programmable data processing apparatus and/or other devices to function in a particular manner, such that the computer readable storage medium having instructions stored therein can comprise an article of manufacture including instructions which can implement aspects of the function/act specified in the flowchart and/or block diagram block or blocks. The computer readable program instructions can also be loaded onto a computer, other programmable data processing apparatus and/or other device to cause a series of operational acts to be performed on the computer, other programmable apparatus and/or other device to produce a computer implemented process, such that the instructions which execute on the computer, other programmable apparatus and/or other device implement the functions/acts specified in the flowchart and/or block diagram block or blocks.

The flowcharts and block diagrams in the figures illustrate the architecture, functionality and/or operation of possible implementations of systems, computer-implementable methods and/or computer program products according to one or more example embodiments described herein. In this regard, each block in the flowchart or block diagrams can represent a module, segment and/or portion of instructions, which comprises one or more executable instructions for implementing the specified logical function. In one or more alternative implementations, the functions noted in the blocks can occur out of the order noted in the Figures. For example, two blocks shown in succession can be executed substantially concurrently, and/or the blocks can sometimes be executed in the reverse order, depending upon the functionality involved. It will also be noted that each block of the block diagrams and/or flowchart illustration, and/or combinations of blocks in the block diagrams and/or flowchart illustration, can be implemented by special purpose hardware-based systems that can perform the specified functions and/or acts and/or carry out one or more combinations of special purpose hardware and/or computer instructions.

While the subject matter has been described above in the general context of computer-executable instructions of a computer program product that runs on a computer and/or computers, those skilled in the art will recognize that the one or more example embodiments herein also can be implemented at least partially in parallel with one or more other program modules. Generally, program modules include routines, programs, components and/or data structures that perform particular tasks and/or implement particular abstract data types. Moreover, the aforedescribed computer-implemented methods can be practiced with other computer system configurations, including single-processor and/or multiprocessor computer systems, mini-computing devices, mainframe computers, as well as computers, hand-held computing devices (e.g., PDA, phone), and/or microprocessor-based or programmable consumer and/or industrial electronics. The illustrated aspects can also be practiced in distributed computing environments in which tasks are performed by remote processing devices that are linked through a communications network. However, one or more, if not all aspects of the one or more example embodiments described herein can be practiced on stand-alone computers. In a distributed computing environment, program modules can be located in both local and remote memory storage devices.

As used in this application, the terms "component," "system," "platform" and/or "interface" can refer to and/or can include a computer-related entity or an entity related to an operational machine with one or more specific functionalities. The entities described herein can be either hardware, a combination of hardware and software, software, or software in execution. For example, a component can be, but is not limited to being, a process running on a processor, a processor, an object, an executable, a thread of execution, a program and/or a computer. By way of illustration, both an application running on a server and the server can be a component. One or more components can reside within a process and/or thread of execution and a component can be localized on one computer and/or distributed between two or more computers. In another example, respective components can execute from various computer readable media having various data structures stored thereon. The components can communicate via local and/or remote processes such as in accordance with a signal having one or more data packets (e.g., data from one component interacting with another component in a local system, distributed system and/or across a network such as the Internet with other systems via the signal). As another example, a component can be an apparatus with specific functionality provided by mechanical parts operated by electric or electronic circuitry, which is operated by a software and/or firmware application executed by a processor. In such a case, the processor can be internal and/or external to the apparatus and can execute at least a part of the software and/or firmware application. As yet another example, a component can be an apparatus that provides specific functionality through electronic components without mechanical parts, where the electronic components can include a processor and/or other means to execute software and/or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Moreover, articles "a" and "an" as used in the subject specification and annexed drawings should generally be construed to mean "one or more" unless specified otherwise or clear from context to be directed to a singular form. As used herein, the terms "example" and/or "exemplary" are utilized to mean serving as an example, instance, or illustration. For the avoidance of doubt, the subject matter described herein is not limited by such examples. In addition, any aspect or design described herein as an "example" and/or "exemplary" is not necessarily to be construed as preferred or advantageous over other aspects or designs, nor is it meant to preclude equivalent exemplary structures and techniques known to those of ordinary skill in the art.

As it is employed in the subject specification, the term "processor" can refer to substantially any computing processing unit and/or device comprising, but not limited to, single-core processors; single-processors with software multithread execution capability; multi-core processors; multi-core processors with software multithread execution capability; multi-core processors with hardware multithread technology; parallel platforms; and/or parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, and/or any combination thereof designed to perform the functions described herein. Further, processors can exploit nanoscale architectures such as, but not limited to, molecular and quantum-dot based transistors, switches and/or gates, in order to optimize space usage and/or to enhance performance of related equipment. A processor can be implemented as a combination of computing processing units.

Herein, terms such as "store," "storage," "data store," data storage," "database," and substantially any other information storage component relevant to operation and functionality of a component are utilized to refer to "memory components," entities embodied in a "memory," or components comprising a memory. Memory and/or memory components described herein can be either volatile memory or nonvolatile memory or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory and/or nonvolatile random-access memory (RAM) (e.g., ferroelectric RAM (FeRAM). Volatile memory can include RAM, which can act as external cache memory, for example. By way of illustration and not limitation, RAM can be available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM) and/or Rambus dynamic RAM (RDRAM). Additionally, the described memory components of systems and/or computer-implemented methods herein are intended to include, without being limited to including, these and/or any other suitable types of memory.

What has been described above includes mere examples of systems and computer-implemented methods. It is, of course, not possible to describe every conceivable combination of components and/or computer-implemented methods for purposes of describing the one or more example embodments, but one of ordinary skill in the art can recognize that many further combinations and/or permutations of the one or more example embodments are possible. Furthermore, to the extent that the terms "includes," "has," "possesses," and the like are used in the detailed description, claims, appendices and/or drawings such terms are intended to be inclusive in a manner similar to the term "comprising" as "comprising" is interpreted when employed as a transitional word in a claim.

The descriptions of the various embodments can use the phrases "an embodiment," "various embodments," "one or more example embodments" and/or "some embodments," each of which can refer to one or more of the same or different embodments.

The descriptions of the various embodments have been presented for purposes of illustration but are not intended to be exhaustive or limited to the embodments described herein. Many modifications and variations will be apparent to those of ordinary skill in the art without departing from the scope and spirit of the described embodments. The terminology used herein was chosen to best explain the principles of the embodments, the practical application and/or technical improvement over technologies found in the marketplace, and/or to enable others of ordinary skill in the art to understand the embodiments described herein.

CLAUSES

Clause 1. A system, comprising: a memory that stores computer executable components; and a processor that executes the computer executable components stored in the memory, wherein the computer executable components comprise: an identifying component that identifies a first set of spectral breakdown data for a first compound, corresponding to first ion activation energies that comprise a target ion activation energy omitted from prior spectrometry measurement of the first compound, wherein the identifying component further identifies a second set of spectral breakdown data for a second compound, corresponding to second ion activation energies; and a comparing component that executes a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

Clause 2. The system of clause 1, wherein the computer executable components further comprise: an approximating component that approximates, using an approximating function, first breakdown curves representing a first set of mass spectrometry data.

Clause 3. The system of clause 1, wherein the computer executable components further comprise: a generating component that generates target spectral breakdown data, of the first set of spectral breakdown data, at the target ion activation energy, and wherein the generating component, based on the target spectral breakdown data, generates target spectrum data defining a target spectrum at the target ion activation energy.

Clause 4. The system of clause 1, wherein the computer executable components further comprise: a generating component that, based on an approximating using an approximating function of first breakdown curves defining a first set of mass spectrometry data, generates the first set of spectral breakdown data for a range of ion activation energies, including the first ion activation energies and additional ion activation energies omitted from the prior spectrometry measurement of the first compound, including the target ion activation energy.

Clause 5. The system of clause 4, wherein the computer executable components further comprise: an outputting component that, based at least on the comparison, generates comparison data comprising terms of ion activation energy per similarity value, including the target similarity value, between the first set of spectral breakdown data and the second set of spectral breakdown data, for a group of fragmentation ions commonly fragmented from the first compound and the second compound.

Clause 6. The system of clause 4, wherein the computer executable components further comprise: an evaluating component that identifies an output ion activation energy, being the target ion activation energy or another ion activation energy, from the range of ion activation energies, as corresponding to a maximum difference in quantified similarity between the first set of spectral breakdown data and the second set of spectral breakdown data; and a notifying component that, in response to an identification of the output ion activation energy, generates a notification requesting re-fragmenting of the first compound at the output ion activation energy.

Clause 7. The system of clause 4, wherein the computer executable components further comprise: an outputting component that, based on the comparison and on additional comparison of the first set of spectral breakdown data to the second set of spectral breakdown data at additional ion activation energies of the range of ion activation energies, generates comparison data comprising terms of ion activation energy per similarity value, including the target similarity value, between the first set of spectral breakdown data and the second set of spectral breakdown data, for the group of fragmentation ions commonly fragmented from the first compound and the second compound, wherein the comparison data comprises total spectra similarity data based on integrals of an analytical functions over the range of ion activation energies as normalized to areas represented by the first breakdown curves defined by the first set of spectral breakdown data and second breakdown curves defined by the second set of spectral breakdown data over the range of ion activation energies.

Clause 8. The system of clause 7, wherein the computer executable components further comprise: a graphing component that generates a node-based graph comprising nodes corresponding to the first compound and the second compound, and edges extending between the nodes and corresponding to a total spectra similarity value, of the total spectra similarity data, between the first compound and the second compound.

Clause 9. The system of clause 7, wherein the total spectra similarity data is further based on a normalization of the range of ion activation energies between a first spectrometry device, at which the prior spectrometry measurement of the first compound was performed, and a second spectrometry device, at which spectrometry measurement of the second compound was performed.

Clause 10. A computer-implemented method, comprising: identifying, by a system operatively coupled to a processor, a first set of spectral breakdown data for a first compound, corresponding to first ion activation energies that comprise a target ion activation energy omitted from prior spectrometry measurement of the first compound; identifying, by the system, a second set of spectral breakdown data for a second compound, corresponding to second ion activation energies; and executing, by the system, a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

Clause 11. The computer-implemented method of clause 10, further comprising: approximating, by the system, using an approximating function, first breakdown curves representing a first set of mass spectrometry data.

Clause 12. The computer-implemented method of clause 11, further comprising: generating, by the system, target spectral breakdown data, of the first set of spectral breakdown data, at the target ion activation energy; and generating, by the system, based on the target spectral breakdown data, target spectrum data defining a target spectrum at the target ion activation energy.

Clause 13. The computer-implemented method of clause 10, further comprising: generating, by the system, based on an approximating using an approximating function of first breakdown curves defining a first set of mass spectrometry data, the first set of spectral breakdown data for a range of ion activation energies, including the first ion activation energies and additional ion activation energies omitted from the prior spectrometry measurement of the first compound, including the target ion activation energy.

Clause 14. The computer-implemented method of clause 13, further comprising: generating, by the system, based at least on the comparison, comparison data comprising terms of ion activation energy per similarity value, including the target similarity value, between the first set of spectral breakdown data and the second set of spectral breakdown data, for a group of fragmentation ions commonly fragmented from the first compound and the second compound.

Clause 15. The computer-implemented method of clause 13, further comprising: identifying, by the system, an output ion activation energy, being the target ion activation energy or another ion activation energy, from the range of ion activation energies, as corresponding to a maximum difference in quantified similarity between the first set of spectral breakdown data and the second set of spectral breakdown data; and generating, by the system, in response to an identification of the output ion activation energy, a notification requesting re-fragmenting of the first compound at the output ion activation energy.

Clause 16. A computer program product facilitating a process for evaluating similarity between spectral datasets, the computer program product comprising a computer readable storage medium having program instructions embodied therewith, and the program instructions executable by a processor to cause the processor to: identify, by the processor, a first set of spectral breakdown data for a first compound, corresponding to first ion activation energies that comprise a target ion activation energy omitted from prior spectrometry measurement of the first compound; identify, by the processor, a second set of spectral breakdown data for a second compound, corresponding to second ion activation energies; and execute, by the processor, a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

Clause 17. The computer program product of clause 16, wherein the program instructions are further executable by the processor to cause the processor to: approximate, by the processor, using an approximating function, first breakdown curves representing a first set of mass spectrometry data.

Clause 18. The computer program product of clause 17, wherein the program instructions are further executable by the processor to cause the processor to: generate, by the processor, target spectral breakdown data, of the first set of spectral breakdown data, at the target ion activation energy; and generate, by the processor, based on the target spectral breakdown data, target spectrum data defining a target spectrum at the target ion activation energy.

Clause 19. The computer program product of clause 16, wherein the program instructions are further executable by the processor to cause the processor to: generate, by the processor, based on an approximating using an approximating function of first breakdown curves defining a first set of mass spectrometry data, the first set of spectral breakdown data for a range of ion activation energies, including the first ion activation energies and additional ion activation energies omitted from the prior spectrometry measurement of the first compound, including the target ion activation energy; and generate, by the processor, based at least on the comparison, comparison data comprising terms of ion activation energy per similarity value, including the target similarity value, between the first set of spectral breakdown data and the second set of spectral breakdown data, for a group of fragmentation ions commonly fragmented from the first compound and the second compound.

Clause 20. The computer program product of clause 15, wherein the program instructions are further executable by the processor to cause the processor to: generate, by the processor, based on an approximating of a first set of mass spectrometry data using an approximating function, the first set of spectral breakdown data for a range of ion activation energies, including the first ion activation energies and additional ion activation energies omitted from the prior spectrometry measurement of the first compound, including the target ion activation energy; identify, by the processor, an output ion activation energy, being the target ion activation energy or another ion activation energy, from the range of ion activation energies, as corresponding to a maximum difference in quantified similarity between the first set of spectral breakdown data and the second set of spectral breakdown data; and generate, by the processor, in response to an identification of the output ion activation energy, a notification requesting re-fragmenting of the first compound at the output ion activation energy.

## Claims

1. A system, comprising:
   a memory that stores computer executable components; and
   a processor that executes the computer executable components stored in the memory, wherein the computer executable components comprise:

   an identifying component that identifies a first set of spectral breakdown data for a first compound, corresponding to first ion activation energies that comprise a target ion activation energy omitted from prior spectrometry measurement of the first compound,
   wherein the identifying component further identifies a second set of spectral breakdown data for a second compound, corresponding to second ion activation energies; and
   a comparing component that executes a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

2. The system of claim 1, wherein the computer executable components further comprise:
   an approximating component that approximates, using an approximating function, first breakdown curves representing a first set of mass spectrometry data.

3. The system of claim 1, wherein the computer executable components further comprise:

   a generating component that generates target spectral breakdown data, of the first set of spectral breakdown data, at the target ion activation energy, and
   wherein the generating component, based on the target spectral breakdown data, generates target spectrum data defining a target spectrum at the target ion activation energy.

4. The system of claim 1, wherein the computer executable components further comprise:
   a generating component that, based on an approximating using an approximating function of first breakdown curves defining a first set of mass spectrometry data, generates the first set of spectral breakdown data for a range of ion activation energies, including the first ion activation energies and additional ion activation energies omitted from the prior spectrometry measurement of the first compound, including the target ion activation energy.

5. The system of claim 4, wherein the computer executable components further comprise:
an outputting component that, based at least on the comparison, generates comparison data comprising terms of ion activation energy per similarity value, including the target similarity value, between the first set of spectral breakdown data and the second set of spectral breakdown data, for a group of fragmentation ions commonly fragmented from the first compound and the second compound.

6. The system of claim 4, wherein the computer executable components further comprise:

an evaluating component that identifies an output ion activation energy, being the target ion activation energy or another ion activation energy, from the range of ion activation energies, as corresponding to a maximum difference in quantified similarity between the first set of spectral breakdown data and the second set of spectral breakdown data; and
a notifying component that, in response to an identification of the output ion activation energy, generates a notification requesting re-fragmenting of the first compound at the output ion activation energy.

7. The system of claim 4, wherein the computer executable components further comprise:

an outputting component that, based on the comparison and on additional comparison of the first set of spectral breakdown data to the second set of spectral breakdown data at additional ion activation energies of the range of ion activation energies, generates comparison data comprising terms of ion activation energy per similarity value, including the target similarity value, between the first set of spectral breakdown data and the second set of spectral breakdown data, for the group of fragmentation ions commonly fragmented from the first compound and the second compound,
wherein the comparison data comprises total spectra similarity data based on integrals of an analytical functions over the range of ion activation energies as normalized to areas represented by the first breakdown curves defined by the first set of spectral breakdown data and second breakdown curves defined by the second set of spectral breakdown data over the range of ion activation energies.

8. The system of claim 7, wherein the computer executable components further comprise:
a graphing component that generates a node-based graph comprising nodes corresponding to the first compound and the second compound, and edges extending between the nodes and corresponding to a total spectra similarity value, of the total spectra similarity data, between the first compound and the second compound.

9. The system of claim 7, wherein the total spectra similarity data is further based on a normalization of the range of ion activation energies between a first spectrometry device, at which the prior spectrometry measurement of the first compound was performed, and a second spectrometry device, at which spectrometry measurement of the second compound was performed.

10. A computer-implemented method, comprising:

identifying, by a system operatively coupled to a processor, a first set of spectral breakdown data for a first compound, corresponding to first ion activation energies that comprise a target ion activation energy omitted from prior spectrometry measurement of the first compound;
identifying, by the system, a second set of spectral breakdown data for a second compound, corresponding to second ion activation energies; and
executing, by the system, a comparison of the first set of spectral breakdown data to the second set of spectral breakdown data at the target ion activation energy, resulting in a target similarity value defining a similarity between the first set of spectral breakdown data and the second set of spectral breakdown data at the target ion activation energy.

11. The computer-implemented method of claim 10, further comprising:
approximating, by the system, using an approximating function, first breakdown curves representing a first set of mass spectrometry data.

12. The computer-implemented method of claim 11, further comprising:

generating, by the system, target spectral breakdown data, of the first set of spectral breakdown data, at the target ion activation energy; and

generating, by the system, based on the target spectral breakdown data, target spectrum data defining a target spectrum at the target ion activation energy.

13. The computer-implemented method of claim 10, further comprising:

generating, by the system, based on an approximating using an approximating function of first breakdown curves defining a first set of mass spectrometry data, the first set of spectral breakdown data for a range of ion activation energies, including the first ion activation energies and additional ion activation energies omitted from the prior spectrometry measurement of the first compound, including the target ion activation energy.

14. The computer-implemented method of claim 13, further comprising:

generating, by the system, based at least on the comparison, comparison data comprising terms of ion activation energy per similarity value, including the target similarity value, between the first set of spectral breakdown data and the second set of spectral breakdown data, for a group of fragmentation ions commonly fragmented from the first compound and the second compound.

15. The computer-implemented method of claim 13, further comprising:

identifying, by the system, an output ion activation energy, being the target ion activation energy or another ion activation energy, from the range of ion activation energies, as corresponding to a maximum difference in quantified similarity between the first set of spectral breakdown data and the second set of spectral breakdown data; and

generating, by the system, in response to an identification of the output ion activation energy, a notification requesting re-fragmenting of the first compound at the output ion activation energy.

FIG. 1

EP 4 745 974 A1

FIG. 2

**EXAMPLE BREAKDOWN CURVE DATA 251A**

FIG. 3

EXAMPLE APPROXIMATING GRAPHS 400 OF EXAMPLE BREAKDOWN DATA 300

FIG. 4

EXAMPLE GRAPH 500 OF APPROXIMATED
SPECTRAL BREAKDOWN DATA 254A

TARGET SPECTRUM 512
at NCE 94

FIG. 5

**EXAMPLE COMPARISON DATA GRAPH 600**

Graph — vertical axis: QUANTIFIED SIMILARITY (1.0, 0.8, 0.6, 0.4, 0.2, 0.0); horizontal axis: ION ACTIVATION ENERGY (NCE) (25, 50, 75, 100, 125, 150, 175, 200).

Labels: 272, 602, 604, 604

**FIG. 6**

**EXAMPLE COMPARISON DATA GRAPH 700**

NOTIFICATION 780

272

714

712

NCE 160

NCE 77

| | |
|---|---|
| [2,3 0] [2,4 0] | Area = 0.8058 |
| [2,3 0] [2,5 0] | Area = 0.8220 |
| [2,3 0] [3,4 0] | Area = 0.9770 |
| [2,3 0] [3,5 0] | Area = 0.7988 |
| [2,4 0] [2,5 0] | Area = 0.6495 |
| [2,4 0] [3,4 0] | Area = 0.7878 |
| [2,4 0] [3,5 0] | Area = 0.9190 |
| [2,5 0] [3,4 0] | Area = 0.8157 |
| [2,5 0] [3,5 0] | Area = 0.6699 |
| [3,4 0] [3,5 0] | Area = 0.8441 |

ION ACTIVATION ENERGY (NCE)

QUANTIFIED SIMILARITY

25 50 75 100 125 150 175 200

0.0 0.2 0.4 0.6 0.8 1.0

**FIG. 7**

FIG. 8

## EXAMPLE COMPARISON DATA GRAPH 900

## SUMMARY OF PROCESSES 950

| MASS SPECTROMETRY DATA 250 | → | BREAKDOWN CURVE DATA 251 | → | SPECTRAL BREAKDOWN DATA 254 | → | TARGET SPECTRUM DATA 510 | → | COMPARISON DATA 272 |
|---|---|---|---|---|---|---|---|---|
| IDENTIFYING COMPONENT 210 | APPROXIMATING COMPONENT 212 | APPROXIMATING COMPONENT 212 (APPROXIMATING 450) | GENERATING COMPONENT 214 | COMPARING COMPONENT 216 (COMPARISON 270) |

FIG. 9

EP 4 745 974 A1

1000

IDENTIFYING, BY THE SYSTEM, A FIRST SET OF SPECTRAL BREAKDOWN DATA FOR A FIRST COMPOUND, CORRESPONDING TO FIRST ION ACTIVATION ENERGIES THAT COMPRISE A TARGET ION ACTIVATION ENERGY OMITTED FROM PRIOR SPECTROMETRY MEASUREMENT OF THE FIRST COMPOUND, AND A SECOND SET OF SPECTRAL BREAKDOWN DATA FOR A SECOND COMPOUND, CORRESPONDING TO SECOND ION ACTIVATION ENERGIES.

1002

NO

IS SUFFICIENTLY EXPANDED DATA PROVIDED TO FACILITATE A COMPARISON?

1004

YES

EXECUTING, BY THE SYSTEM, A COMPARISON OF THE FIRST SET OF SPECTRAL BREAKDOWN DATA TO THE SECOND SET OF SPECTRAL BREAKDOWN DATA AT THE TARGET ION ACTIVATION ENERGY, RESULTING IN A TARGET SIMILARITY VALUE DEFINING A SIMILARITY BETWEEN THE FIRST SET OF SPECTRAL BREAKDOWN DATA AND THE SECOND SET OF SPECTRAL BREAKDOWN DATA AT THE TARGET ION ACTIVATION ENERGY.

1006

END

# FIG. 10

1100

| 1102 | IDENTIFYING, BY A SYSTEM COUPLED TO A PROCESSOR, A FIRST SET OF MASS SPECTROMETRY DATA. |

| 1104 | COMPRISE IDENTIFYING, BY THE SYSTEM, A FIRST SET OF SPECTRAL BREAKDOWN DATA FOR A FIRST COMPOUND, CORRESPONDING TO FIRST ION ACTIVATION ENERGIES THAT COMPRISE A TARGET ION ACTIVATION ENERGY OMITTED FROM PRIOR SPECTROMETRY MEASUREMENT OF THE FIRST COMPOUND, AND A SECOND SET OF SPECTRAL BREAKDOWN DATA FOR A SECOND COMPOUND, CORRESPONDING TO SECOND ION ACTIVATION ENERGIES. |

| 1106 | APPROXIMATING, BY THE SYSTEM, USING AN APPROXIMATING FUNCTION, FIRST BREAKDOWN CURVES REPRESENTING THE FIRST SET OF MASS SPECTROMETRY DATA. |

| 1108 | GENERATING, BY THE SYSTEM, TARGET SPECTRAL BREAKDOWN DATA, OF THE FIRST SET OF SPECTRAL BREAKDOWN DATA, AT A TARGET ION ACTIVATION ENERGY. |

| 1110 | GENERATING, BY THE SYSTEM, BASED ON THE TARGET SPECTRAL BREAKDOWN DATA, TARGET SPECTRUM DATA DEFINING A TARGET SPECTRUM AT THE TARGET ION ACTIVATION ENERGY. |

| 1112 | GENERATING, BY THE SYSTEM, BASED ON THE APPROXIMATING USING THE APPROXIMATING FUNCTION OF THE FIRST BREAKDOWN CURVES DEFINING THE FIRST SET OF MASS SPECTROMETRY DATA, GENERATES THE FIRST SET OF SPECTRAL BREAKDOWN DATA FOR A RANGE OF ION ACTIVATION ENERGIES, INCLUDING THE FIRST ION ACTIVATION ENERGIES AND ADDITIONAL ION ACTIVATION ENERGIES OMITTED FROM THE PRIOR SPECTROMETRY MEASUREMENT OF THE FIRST COMPOUND, INCLUDING THE TARGET ION ACTIVATION ENERGY. |

| 1114 | EXECUTING, BY THE SYSTEM, A COMPARISON OF THE FIRST SET OF SPECTRAL BREAKDOWN DATA TO THE SECOND SET OF SPECTRAL BREAKDOWN DATA AT THE TARGET ION ACTIVATION ENERGY, RESULTING IN A TARGET SIMILARITY VALUE DEFINING A SIMILARITY BETWEEN THE FIRST SET OF SPECTRAL BREAKDOWN DATA AND THE SECOND SET OF SPECTRAL BREAKDOWN DATA AT THE TARGET ION ACTIVATION ENERGY. |

A

**FIG. 11**

FIG. 12

B

1100

1128 — IDENTIFYING, BY THE SYSTEM, AN OUTPUT ION ACTIVATION ENERGY, BEING THE TARGET ION ACTIVATION ENERGY OR ANOTHER ION ACTIVATION ENERGY, FROM THE RANGE OF ION ACTIVATION ENERGIES, AS CORRESPONDING TO A MAXIMUM DIFFERENCE IN QUANTIFIED SIMILARITY BETWEEN THE FIRST SET OF SPECTRAL BREAKDOWN DATA AND THE SECOND SET OF SPECTRAL BREAKDOWN DATA.

1130 — GENERATING, BY THE SYSTEM, IN RESPONSE TO AN IDENTIFICATION OF THE OUTPUT ION ACTIVATION ENERGY, A NOTIFICATION REQUESTING RE-FRAGMENTING OF THE FIRST COMPOUND AT THE OUTPUT ION ACTIVATION ENERGY.

END

# FIG. 13

1400

1410
REMOTE
COMPONENT(S)

1420
LOCAL
COMPONENT(S)

REMOTE
DATA
STORE(S)
1450

COMMUNICATION
FRAMEWORK

LOCAL
DATA
STORE(S)
1430

1440

FIG. 14

1500

1502

PROCESSING
UNIT — 1504

1508   1506

SYSTEM
MEMORY
— 1512

RAM

ROM — 1510

1524

INTERFACE

1526

INTERFACE

1528

INTERFACE

VIDEO
ADAPTOR — 1548

(WIRED/WIRELESS)

INPUT
DEVICE
INTERFACE — 1544

NETWORK
ADAPTOR — 1558

(WIRED/WIRELESS)

BUS

1530
OPERATING SYSTEM

1532
APPLICATIONS

1534
MODULES

1536
DATA

HDD — 1514

EXTERNAL
STORAGE — 1516

Storage
Device — 1520

MEDIA — 1522

MONITOR — 1546

KEYBOARD — 1538

TOUCH
SCREEN — 1540

MOUSE — 1542

MODEM — 1560

WAN — 1556

LAN — 1554

REMOTE
COMPUTER(S) — 1550

MEMORY/
STORAGE — 1552

**FIG. 15**

Europäisches
Patentamt

European
Patent Office

Office européen
des brevets

# EUROPEAN SEARCH REPORT

**Application Number**

EP 25 21 5665

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KING ETHAN ET AL: "Augmentation of MS/MS Libraries with Spectral Interpolation for Improved Identification", JOURNAL OF CHEMICAL INFORMATION AND MODELING, vol. 62, no. 16, 29 July 2022 (2022-07-29), pages 3724-3733, XP093356282, US ISSN: 1549-9596, DOI: 10.1021/acs.jcim.2c00620 Retrieved from the Internet: URL:https://pubs.acs.org/doi/pdf/10.1021/acs.jcim.2c00620> | 1-5,7,8, 10-14 | INV. G16C20/20 H01J49/00 |
| A | * the whole document * | 6,9,15 | |
| A | WO 2014/096915 A1 (DH TECHNOLOGIES DEV PTE LTD [SG]) 26 June 2014 (2014-06-26) * the whole document * | 1-15 | |
| A | KOS KUKI ET AL: "Collision induced dissociation study of the major components of silymarin", INTERNATIONAL JOURNAL OF MASS SPECTROMETRY ELSEVIER SCIENCE PUBLISHERS , AMSTERDAM, NL, vol. 315, 24 February 2012 (2012-02-24), pages 46-54, XP028405843, ISSN: 1387-3806, DOI: 10.1016/J.IJMS.2012.02.021 [retrieved on 2012-03-22] * the whole document * | 1-15 | TECHNICAL FIELDS SEARCHED (IPC) G16C F03H H01J |

-/--

The present search report has been drawn up for all claims

| Place of search | Date of completion of the search | Examiner |
|---|---|---|
| The Hague | 30 March 2026 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 25 21 5665

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MATTHEW J. CARLO ET AL: "Further exploration of the collision-induced dissociation of select beta blockers: Acebutolol, atenolol, bisoprolol, carteolol, and labetalol", JOURNAL OF MASS SPECTROMETRY WILEY CHICHESTER, GB, vol. 58, no. 12, 21 November 2023 (2023-11-21), pages n/a-n/a, XP072549367, ISSN: 1076-5174, DOI: 10.1002/JMS.4985 * the whole document * ----- | 1-15 | |

|  |  |  | TECHNICAL FIELDS SEARCHED (IPC) |
|---|---|---|---|

The present search report has been drawn up for all claims

| Place of search | Date of completion of the search | Examiner |
|---|---|---|
| The Hague | 30 March 2026 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 2 of 2

## ANNEX TO THE EUROPEAN SEARCH REPORT
## ON EUROPEAN PATENT APPLICATION NO.

EP 25 21 5665

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2026

| Patent document cited in search report | Publication date | Patent family member(s) | Publication date |
|---|---|---|---|
| WO 2014096915 A1 | 26-06-2014 | CN 104798174 A | 22-07-2015 |
| | | EP 2936544 A1 | 28-10-2015 |
| | | WO 2014096915 A1 | 26-06-2014 |

EPO FORM P0459

For more details about this annex : see Official Journal of the European Patent Office, No. 12/82

**EP 4 745 974 A1**

**REFERENCES CITED IN THE DESCRIPTION**

**Patent documents cited in the description**

- US 94753124